(19) Europäisches Patentamt

European Patent Office

Office européen des brevets

(11) **EP 1 035 866 B1**

(12) **FASCICULE DE BREVET EUROPEEN**

(45) Date de publication et mention
de la délivrance du brevet:
**17.05.2006 Bulletin 2006/20**

(51) Int Cl.:
*A61K 39/39* (2006.01)   *A61K 9/107* (2006.01)

(21) Numéro de dépôt: **98958314.1**

(22) Date de dépôt: **02.12.1998**

(86) Numéro de dépôt international:
**PCT/FR1998/002605**

(87) Numéro de publication internationale:
**WO 1999/027954 (10.06.1999 Gazette 1999/23)**

(54) **MICELLES MIXTES DE LIPOPEPTIDES POUR L'INDUCTION D'UNE REPONSE IMMUNITAIRE**

MISCMICELLEN AUS LIPOPEPTIDEN FÜR INDUZIERUNG EINER IMMUNANTWORT

MIXED LIPOPEPTIDE MICELLES FOR INDUCING AN IMMUNE RESPONSE AND THEIR THERAPEUTIC USES

(84) Etats contractants désignés:
**AT BE CH DE DK ES FI FR GB GR IE IT LI LU MC NL PT SE**

(30) Priorité: **03.12.1997 FR 9715246**

(43) Date de publication de la demande:
**20.09.2000 Bulletin 2000/38**

(73) Titulaires:
• **INSTITUT NATIONAL DE LA SANTE ET DE LA RECHERCHE MEDICALE (INSERM)**
**75654 Paris Cédex 13 (FR)**
• **CENTRE NATIONAL DE LA RECHERCHE SCIENTIFIQUE**
**75016 Paris Cédex (FR)**
• **INSTITUT PASTEUR DE LILLE**
**59019 Lille Cédex (FR)**

(72) Inventeurs:
• **GRAS-MASSE, Hélène**
**F-59710 Merignies (FR)**
• **BOSSUS, Marc**
**F-59000 Lille (FR)**
• **LIPPENS, Guy**
**F-59211 Santes (FR)**
• **WIERUSZESKI, Jean-Michel**
**F-62221 Noyelles sous Lens (FR)**
• **TARTAR, André**
**F-62490 Vitry en Artois (FR)**
• **GUILLET, Jean-Gérard**
**F-92170 Vanves (FR)**
• **BOURGAULT-VILLADA, Isabelle**
**F-75007 Paris (FR)**

(74) Mandataire: **Michelet, Alain et al**
**Cabinet Harlé et Phélip**
**7, rue de Madrid**
**75008 Paris (FR)**

(56) Documents cités:
**EP-A- 0 491 628**   **WO-A-96/17863**
**WO-A-96/40213**

• **BENMOHAMED L ET AL: "Lipopeptide immunization without adjuvant induces potent and long-lasting B, T helper, and cytotoxic T lymphocyte responses against a malaria liver stage antigen in mice and chimpanzees."** EUROPEAN JOURNAL OF IMMUNOLOGY, (1997 MAY) 27 (5) 1242-53, XP002075035
• **LIVINGSTON B D ET AL: "The hepatitis B virus-specific CTL responses induced in humans by lipopeptide vaccination are comparable to those elicited by acute viral infection."** JOURNAL OF IMMUNOLOGY, (1997 AUG 1) 159 (3) 1383-92, XP002075036
• **DEPREZ B ET AL: "Comparative efficiency of simple lipopeptide constructs for in vivo induction of virus-specific CTL."** VACCINE, (1996 APR) 14 (5) 375-82, XP002075037 cité dans la demande
• **VITIELLO A ET AL: "Development of a lipopeptide-based therapeutic vaccine to treat chronic HBV infection. I. Induction of a primary cytotoxic T lymphocyte response in humans."** JOURNAL OF CLINICAL INVESTIGATION, (1995 JAN) 95 (1) 341-9, XP002075038 cité dans la demande

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

• JEFF A.: 'Development of High Potency Universal DR-Restricted Helper Epitopes by Modification of High Affinity DR-Blocking Peptides' IMMUNITY vol. 1, Décembre 1994, pages 751 - 761

• JEFF A.: 'Development of High Potency Universal DR-Restricted Helper Epitopes by Modification of High Affinity DR-Blocking Peptides' IMMUNITY vol. 1, Décembre 1994, pages 751 - 761

**Description**

La présente demande a pour objet des micelles mixtes de lipopeptides pour l'induction d'une réponse immunitaire.

Elle a en outre pour objet l'utilisation de ces micelles à des fins thérapeutiques.

Il existe deux types de réponse immunitaire effectrice: la réponse humorale due aux anticorps, et la réponse cytotoxique due aux lymphocytes T CD8[+].

Une réponse cytotoxique efficace requiert la présentation des antigènes aux lymphocytes T cytotoxiques CD8[+] (CTL), en association avec les molécules de classe I du Complexe Majeur d'Histocompatibilité (MHC), mais aussi aux lymphocytes T auxiliaires CD4[+] (HTL) en association avec les molécules de classe II du MHC.

L'utilisation de lipopeptides pour l'induction d'une réponse cytotoxique, c'est-à-dire la génération in vivo de lymphocytes T cytotoxiques a déjà été décrite. En particulier, la demande FR-90 15 870 publiée sous le n°2 670 787 (Institut Pasteur de Lille, Institut Pasteur, INSERM) décrit des lipopeptides constitués d'une partie peptidique comprenant de 10 à 40 acides aminés et d'une partie lipidique qui peut être dérivée d'acides gras ou de groupements stéroïdes.

Ces lipopeptides montrent une bonne aptitude à induire une réponse cytotoxique. Il convenait cependant de les rendre capables d'induire une réponse de meilleure qualité par l'adjonction d'une réponse T auxiliaire dont on sait l'importance pour l'induction efficace et le maintien de la réponse cytotoxique. Il convenait en outre de les rendre capable d'induire une réponse chez le plus grand nombre d'individus.

**BOURGAULT et al.** (1994,J. Immunol., 2530-2537) ont induit une réponse CTL et HTL à partir de mélange de lipopeptides, sous la forme d'une émulsion avec un adjuvant huileux.

Néanmoins, il était nécessaire d'ajouter de l'adjuvant incomplet de Freund (IFA). L'immunogénicité de la préparation vaccinale utilisée impliquait nécessairement la co-présentation fonctionnelle des motifs HTL et CTL situés dans un ou plusieurs lipopeptides du mélange. Cependant, l'efficacité de la co-présentation des différents motifs impliqués dépendait de l'association avec l'adjuvant Incomplet de Freund au sein d'une émulsion très fine.

Un article au nom de **VITIELLO et al.** (1995, J. Clin. Invest.. 95, 341-349) évoque la possibilité d'induire une réponse CTL dans une population humaine sélectionnée (HLA-A2) en utilisant un lipopeptide comportant une association séquentielle entre un épitope CTL HLA-A2 et un épitope auxiliaire (HTL) multivalent. On notera que cette étude a été menée sur une population génétiquement restreinte.

Cet article rend compte également d'une expérience au cours de laquelle deux types d'associations entre épitope HTL et épitope CTL ont été comparées: d'une part une association covalente séquentielle au sein d'un même lipopeptide, et d'autre part une association par simple mélange d'un lipopeptide comportant le motif CTL avec un peptide comportant le motif HTL. Les résultats de cette étude montrent un avantage très net de l'association covalente par rapport au mélange, tel qu'il a été réalisé par ces auteurs, c'est-à-dire par mélange de solutions comportant du DMSO et du tampon PBS (les peptides ou lipopeptides sont maintenus en solutions stock à une concentration de 10 - 20 mglml et dilués extemporanément avec du PBS avant usage).

Cependant, la combinaison sur une même molécule lipopeptidique des épitopes cytotoxique et auxiliaire, bien que pouvant induire une réponse immunitaire efficace, nécessite la synthèse de séquences d'acides aminés longues présentant de multiples épitopes susceptibles de s'associer à plusieurs HLA ou superfamilles d'HLA de classe I et de classe II. L'association covalente de tous ces motifs au sein d'une unique molécule représente des difficultés techniques difficilement surmontables, tant du point de vue des méthodes de synthèse que des méthodes de caractérisation analytique.

En tout état de cause , cet article mentionne l'association d'un lipopeptide et d'un peptide, et non de deux lipopeptides. De ce fait aucune formation de micelles mixtes ne peut avoir lieu.

Un autre article, publié par **DON DIAMOND et al** (1997, Blood; 90, n°5), mentionne l'immunogénicité d'un mélange entre un peptide portant un épitope CTL minimal (pp65, séquence 495-503 de la protéine de matrice du cytomégalovirus) et un peptide dipalmitoyle contenant un épitope HTL. Le mélange est réalisé par mélange des solutions dans l'acide acétique dilué ou dans le DMSO, en utilisant un traitement par les ultrasons pendant 15 à 30 secondes.

Cet article ne décrit donc pas de mélange de lipopeptides contenant indépendamment un épitope CTL et un épitope HTL, mais le mélange d'un lipopeptide contenant un épitope HTL et d'un nonapeptide correspondant à un épitope CTL minimal. D'ailleurs il n'est pas mentionné de formation de micelles mixtes ou de micro-agrégats. Dans ce cas particulier cependant, la possibilité d'association directe du nonapeptide avec les CMH de classe I exprimés à la surface des cellules peut expliquer le succès de l'approche suivie. L'immunogénicité de la préparation signifie qu'il y a eu effectivement co-présentation des épitopes HTL et CTL par la même cellule présentatrice de l'antigène: cependant, le nonapeptide minimal utilisé présente la capacité à se lier directement avec les CMH de classe I à la surface de la cellule présentatrice de l'antigène, sans que soit nécessaire son apprêtement par la cellule.

Les auteurs concluent en reconnaissant que plusieurs obstacles existent encore pour obtenir une immunité à long terme, ce qui confirme ie caractère expérimental de cette étude.

La difficile obtention d'une réponse immune dépendant d'un double apprêtement de peptides présentant séparément les épitopes HTL et CTL, est maintenant expliquée par une publication de STUHLER (1997, Proc. Natl. Acad. Sci. USA, 94, 622-627). Pour pouvoir observer l'induction d'une réponse CTL, il est absolument nécessaire que les épitopes HTL et CTL soient présentés à la surface de la même cellule présentatrice de l'antigène (CPA) pour pouvoir être reconnu à la fois par les cellules T auxiliaire reconnaissant l'épitope HTL et les cellules T cytotoxiques reconnaissant l'épitope CTL.

Il ressort donc de ce qui précède que des compositions contenant au sein de mêmes micelles, ou de mêmes micro-agrégats, d'une part des lipopeptides présentant un épitope CTL et d'autre part des lipopeptides comprenant un épitope T auxiliaire, c'est-à-dire des micelles ou micro-agrégats mixtes, n'ont à la connaissance du demandeur, jamais été décrites.

Or, comme indiqué ci-dessus, il est absolument nécessaire que les deux épitopes, cytotoxique et T auxiliaire, soient présentés à la surface de la même cellule présentatrice de l'antigène.

Outre la nécessité d'une co-présentation des deux épitopes à la surface de la même cellule, il est aussi indispensable de solubiliser les lipopeptides, afin de permettre leur administration aux patients, et leur stérilisation par filtration.

Le demandeur s'est donc attaché à trouver une solution à ces divers problèmes.

Il a montré que pour obtenir des micelles formées individuellement de tous les peptides présents dans le mélange, qu'ils contiennent des épitopes HTL ou CTL, il était nécessaire d'associer les différents lipopeptides après les avoir au préalable dispersés au stade moléculaire dans un solvant adapté.

La présente invention a donc pour objet des micelles ou micro-agrégats pour l'induction d'une réponse immunitaire contenant au moins:

- un premier lipopeptide comprenant au moins un épitope CTL, ou épitope cytotoxique, et au moins un motif lipidique, et
- un second lipopeptide comprenant au moins un épitope auxiliaire et au moins un motif lipidique, dont la nature peut être différente du motif du premier lipopeptide.

Au sens de la présente invention, l'expression « réponse immunitaire » désigne ia totalité de la réponse immunitaire induite, qui comprend la réponse cytotoxique et la réponse humorale.

Les micelles selon la présente invention ne sont pas limitées à deux lipopeptides, mais peuvent comprendre d'autres lipopeptides présentant indépendamment des épitopes HTL ou CTL.

On entend, pour la compréhension de la présente invention, par épitope T auxiliaire, une séquence d'acides aminés capable de s'associer à au moins un récepteur HLA de classe II, et capable d'être reconnue par les lymphocytes T auxiliaires.

On entend par épitope CTL une séquence d'acides aminés capable de s'associer à au moins un récepteur HLA de classe 1 et capable d'être reconnue par les lymphocytes T cytotoxiques.

Les épitopes T auxiliaires capables de s'associer à plusieurs récepteurs HLA de classe II différents sont appelés épitopes auxiliaires multivalents (HTL multivalents).

En outre, on entend par micelles ou par micro-agrégats des agrégats de lipopeptides présentant une taille les rendant assimilables simultanément par toute cellule présentatrice de l'antigène (CPA) et préférentiellement d'une taille inférieure à environ $1\mu m$.

Les micelles mixtes selon la présente invention, c'est-à-dire comprenant des lipopeptides comportant des épitopes cytotoxiques et des lipopeptides comportant des épitopes T auxiliaires, présentent l'avantage d'associer au sein du même microvolume assimilable par une seule CPA une grande diversité d'épitopes CTL et HTL. sans que soit nécessaire leur association covalente, tout en respectant le critère de définition chimique requis. Des micelles constituées chacune d'une seule variété de lipopeptide, contenant un épitope CTL ou un épitope HTL ne permettent pas d'obtenir une coprésentation efficace correspondant à l'induction d'une réponse effectrice forte.

En outre, l'obtention d'une réponse CTL grâce à l'emploi de micro-agrégats ou de micelles mixtes permet d'éviter l'utilisation d'émulsions avec des adjuvants huileux, tels que l'adjuvant incomplet de Freund, dont l'emploi n'est pas admis en thérapeutique humaine. Les micelles et micro-agrégats selon la présente invention sont cependant compatibles avec l'emploi d'émulsions avec des excipients huileux cliniquement acceptables.

Un autre avantage des micro-agrégats ou micelles mixtes selon la présente invention, comprenant individuellement au moins deux types de lipopeptides, réside dans le fait que la solubilisation de lipopeptides présentant une faible solubilité dans l'eau ou dans des solvants cliniquement acceptables, ou de lipopeptides insolubles, peut être facilitée par leur combinaison avec d'autre(s) lipopeptide(s) présentant une meilleure solubilité.

Les micelles selon la présente invention présentent encore l'avantage, par rapport aux lipopeptides dans lesquels les motifs HTL et CTL sont associés de manière covalente et dont la taille est limitée, tels que décrits par **VITIELLO et al.** (1995, précédemment cité), de permettre l'association d'une grande diversité de motifs et donc de pouvoir être utilisés pour la vaccination de populations humaines ou animales non sélectionnées sur la base de la restriction génétique.

Les micelles selon la présente invention peuvent comprendre un lipopeptide présentant au moins un épitope CTL et un autre lipopeptide comprenant au moins un épitope auxiliaire. Néanmoins, de telles micelles peuvent aussi contenir plusieurs lipopeptides différents comprenant différents épitopes cytotoxiques et différents lipopeptides présentant différents épitopes auxiliaires.

Les motifs lipidiques des lipopeptides peuvent être indépendamment un ou plusieurs motifs en $C_4$ à $C_{18}$, et en particulier une ou plusieurs chaînes dérivées d'acides gras, ou d'alcools gras, en $C_4$ à $C_{18}$, éventuellement ramifiés et insaturés ou être dérivés d'un stéroïde.

Ils peuvent comprendre une ou deux chaînes lipidiques en $C_4$ à $C_{18}$ associées par liaison covalente à un ou deux acides aminés de la partie peptidique. Ils peuvent ainsi être constitués de deux chaînes d'acide palmitique liées aux groupements $NH_2$, alpha et epsilon, d'une lysine.

Ces motifs lipidiques peuvent aussi être constitués de, ou comprendre un résidu d'acide palmitique, d'acide 2-amino-hexadécanoïque d'acide oléique, d'acide linoléique, d'acide linolénique, de pimélautide, de triméxautide, ou un dérivé du cholestérol, ou tout autre constituant lipidique naturel des membranes cellulaires.

De manière avantageuse, les lipopeptides constituant les micelles ou les micro-agrégats mixtes sont hydrosolubles dans une proportion d'au moins 30% (en poids). Ces lipopeptides hydrosolubles ont des propriétés de tensioactifs cationiques, propres à exercer un effet solubilisant vis-à-vis d'autres lipopeptides en milieu acide faible.

La partie non lipidique comprend quant à elle entre 10 et 100, et préférentiellement entre 10 et 50 acides aminés. Le nombre d'acides aminés dépend du nombre d'épitopes constituant la partie non lipidique du lipopeptide et de leurs tailles, de la nature de la partie lipidique, et des proportions des parties lipidiques et non lipidiques,

De manière avantageuse, les épitopes HTL et CTL employés sont des épitopes capables de s'associer à plusieurs HLA différents, ou épitopes multivalents (« promiscuous épitopes » en anglais).

L'épitope HTL employé est préférentiellement constitué par le peptide multivalent 830-843 de la toxine tétanique.

**QYIKANSKFIGITE**

La glutamine (Q) de cette séquence peut éventuellement être acétylée.

D'autres épitopes HTL multivalents peuvent être l'épitope multivalent de l'hémagglutinine (**PREVOST-BLONDEL et al.** 1995, J. Virol., vol.62, n°12, pages 8046-8055) ou encore l'épitope PADRE de séquence AKFVAAWTLKAAA (**ALEXANDER et al.,** 1994, Immunity, 1, 751).

L'épitope CTL peut quant à lui être tout épitope capable d'activer des lymphocytes T cytotoxiques CD8[+].

Il est préférentiellement un épitope CTL d'une protéine présentée par une cellule tumorale et en particulier par un mélanome, d'une protéine du VIH, du virus de l'hépatite B (VHB) ou du papillomavirus, ou encore de la protéine p53.

Il peut être en particulier l'un des épitopes suivants:

- épitopes de la protéine BCR-ABL, résultant de la translocation BCR- Abelson (leucémie myéloïde chronique) tels que mentionnés dans le tableau 1.
- épitopes de la protéine p53, tels que ceux mentionnés dans le tableau 2.

Les épitopes de la protéine p53 peuvent en outre être pris dans les séquences 25-35, 63-73,129-156, 149-156, 187-205, 187-234, 226-264 ou 249-264 de cette protéine.

- épitopes des protéines $E_6$ ou $E_7$ du papillomavirus humain (VPH), tels que ceux mentionnés dans le tableau 3.
- épitopes de protéines du virus VIH-1 tels que ceux mentionnés dans le tableau 4,
- épitopes du mélanome ou d'autres tumeurs, tels que ceux mentionnés dans les tableaux 5, 6 et 7 et en particulier épitopes de l'antigène melan-A/mart-1 du mélanome.

D'autres épitopes CTL plurivalents présentant une capacité d'association avec des HLA de classe 1 peuvent être ceux compris dans le peptide 43-57 de HPV (GQAEPDRAHNIVTF) qui contient des épitopes HLA A2, A24, B7 et B18.

Les épitopes CTL peuvent encore être ceux d'antigènes parasitaires, et en particulier de *Plasmodium falciparum.*

Les micro-agrégats ou micelles mixtes de lipopeptides selon la présente invention peuvent être lyophilisés, puis repris par tout tampon cliniquement acceptable pour être administrés aux patients à traiter, en particulier aux personnes à vacciner.

Ils peuvent être administrés par toute voie d'administration employée en thérapeutique et à titre non limitatif, par voie parentérale, percutanée, orale, sublinguale ou par nébulisat intra-pulmonaire.

Un autre objet de la présente invention est donc l'utilisation de ces lipopeptides pour la fabrication d'un médicament ou d'un vaccin pour l'induction d'une réponse immunitaire spécifique, et en particulier, pour l'induction d'une réponse immunitaire à l'encontre de cancers tels que le mélanome, des virus VIH et VHB, des papillomavirus, de la p53 ou de la malaria.

La présente demande a encore pour objet une composition pharmaceutique caractérisée en ce qu'elle contient une quantité pharmacologiquement active d'un ou plusieurs des lipopeptides décrits ci-dessus, ainsi que des excipients pharmaceutiquement compatibles.

Les lipopeptides formant les micelles selon la présente invention peuvent être fabriqués par tout procédé adéquat connu de l'homme du métier. Ils peuvent en particulier être obtenus par les méthodes Boc-benzyle ou Fmoc-tertiobutyle, en particulier comme décrits dans la demande FR-90 15 870, dont le contenu est intégré par référence à la présente demande .

L'introduction de la chaîne lipidique peut être réalisée en phase solide, après déprotection sélective de la ou les fonction(s) concernées, comme décrit dans l'article de **DEPREZ et al.** (1996 , Vaccine, volume 14, n°5,375-382). La chaîne lipidique peut être introduite sur la fonction $\varepsilon$-NH$_2$ d'une lysine protégée sur la fonction $\alpha$-NH$_2$ par un groupe F-moc. La Fmoc-lys (Palm) obtenue peut être ensuite utilisée en synthèse en phase solide pour élaborer le lipopeptide.

Les micelles et micro-agrégats selon la présente invention peuvent être obtenus en dispersant chaque lipopeptide dans une solution d'acide acétique concentrée à environ 80% puis en mélangeant les solutions ainsi obtenues.

La qualité de la dissolution, c'est à dire la dispersion effective à l'état moléculaire de chaque lipopeptide avant la préparation du mélange est vérifiée par la méthode de résonance magnétique nucléaire en deux dimensions (RMN2D). La résolution du signal obtenu lors d'expériences en deux dimensions homonucléaires dans un champ de 600 Mhz confirme la dispersion complète, au stade moléculaire, des lipopeptides en solution. La limpidité du mélange n'est pas un critère suffisant : en particulier, la reprise des lipopeptides par du DMSO ou un mélange DMSO/eau ne permet pas, dans la plupart des cas, d'obtenir cet état de dispersion, ce qui explique l'inefficacité du mélange étudié par **VITIELLO et al.** (1995, précédemment cité). La dissolution par des mélanges acide acétique/eau plus dilués en acide acétique ne permet pas non plus de préparer dans tous les cas un mélange de micro-agrégats ou micelles mixtes comportant une proportion statistique de chaque constituant du mélange au stade du microvolume. Dans ces deux cas, même en présence d'un mélange apparemment limpide, la filtration stérilisante sur membrane de 0,22 $\mu$m est soit impossible, soit irrégulière, avec des rendements de filtration différents selon les constituants, ce qui signifie qu'à l'échelle d'une particule de cette taille, la représentation de chacun des constituants du mélange n'est pas obtenue. Cette microhétérogénéité compromet l'immunogénicité du mélange, puisqu'elle compromet la capture et la présentation simultanée de tous les constituants par une seule cellule présentatrice d'antigène (CPA), dans le cas d'épitopes CTL et HTL présents sur des lipopeptides distincts.

La présente invention est illustrée, sans pour autant être limitée, par les exemples qui suivent.

La figure 1 représente la structure chimique de la résine de type KNORR-MBHA.

Les figures 2 et 3 représentent des spectres de résonance magnétique nucléaire en deux dimensions (RMN 2D), respectivement d'un lipopeptide seul (lipopeptide ENV), et d'un mélange de lipopeptides.

La figure 4 illustre la réponse auxiliaire de huit macaques immunisés avec un mélange de lipopeptides.

Les figures 5A à 5F illustrent la réponse cytotoxique du macaque n°109.

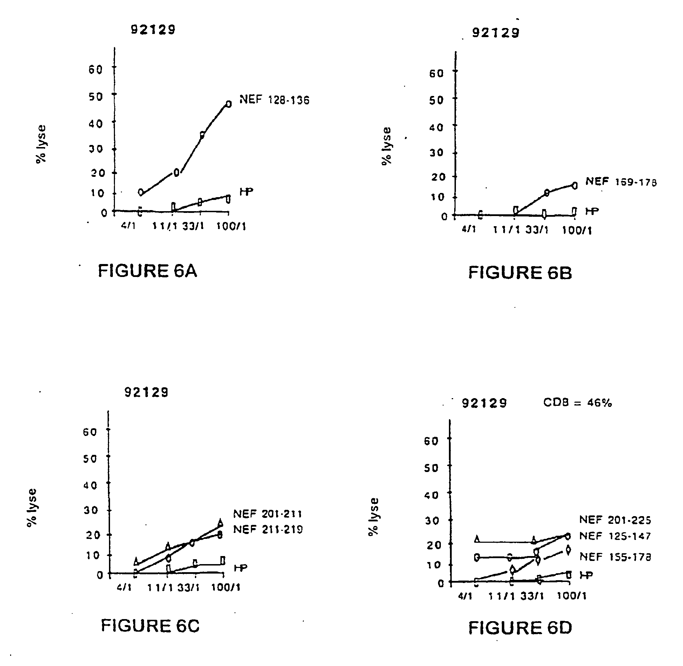

Les figures 6A à 6D illustrent la réponse cytotoxique du macaque n° 129.

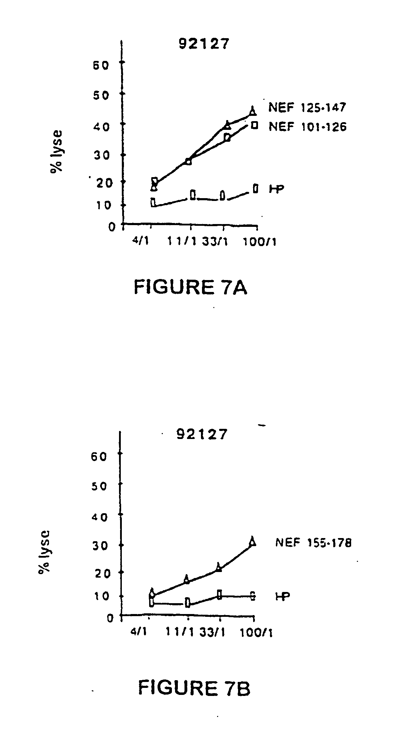

Les figures 7A et 7B illustrent la réponse cytotoxique du macaque n°127.

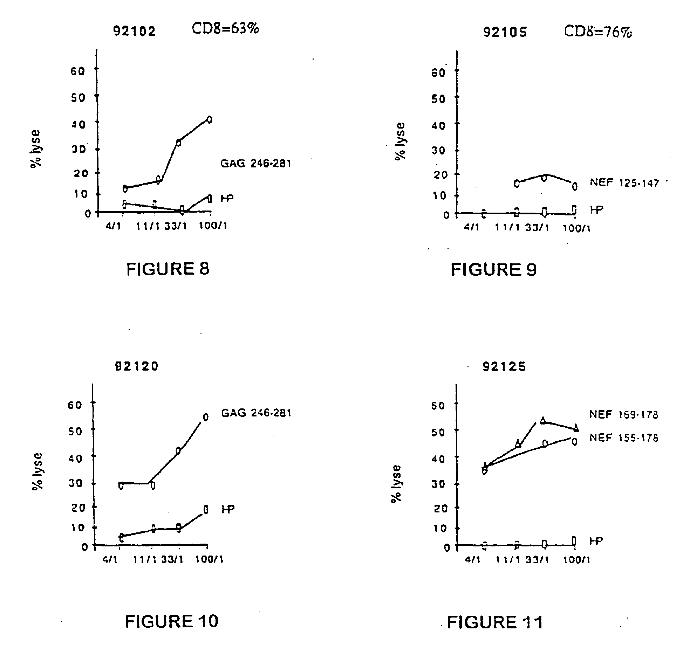

Les figures 8, 9, 10 et 11 illustrent respectivement les réponses cytotoxiques des macaques N[OS] 102, 105, 120 et 125.

Les figures 12A, 12B et 12C représentent respectivement les activités cytolytiques anti-N1, anti-G2 et anti-E des PBMC, de cellules CD8[+] et de cellules CD4[-] de l'individu V4.1.

La figure 13 quant à elle illustre l'activité cytolytique de PBMC de l'individu V4.5 collectées vingt semaines après le début de l'immunisation, stimulées in vitro avec le peptide N2 puis testées pour leur activité CTL à l'encontre de la vaccine sauvage (WT), ou de ce même virus exprimant une protéine NEF recombinante (NEF, NEF-2, NEF-MN, NEF-A, NEF-ROD).

**EXEMPLE 1: Préparation de micelles ou micro-agrégats selon l'invention:**

**1 - description des lipopeptides employés dans le mélange**

| nom | formules: | poids moléculaire |
|---|---|---|
| NEF 66 | V G F P V T P Q V P L R P M T Y K A A V D L S H F L K E K G G L K(Pam)-NH$_2$ | 3862,77 |
| NEF117 | T Q G Y F P D W Q N Y T P G P G V R Y P L T F G W C Y K L V P K(Pam)-NH$_2$ | 4017,754 |
| NEF182 | E W R F D S R L A F H H V AR EL H P E Y F K N K(Pam)-NH$_2$ | 3451,04 |
| GAG 183 NH$_2$ | D L N T M L N T V G G H Q A A M Q M L K E T I N E E A A E W D R K(Pam)- | 3983,65 |
| GAG 253 | N P P I P V G E I Y K R W I I L G L N K I V R M Y S P T S I L D K (Pam)-NH$_2$- | 4063,05 |
| ENV | T R P N N N T R K S I H G P G R A F Y A T G E I I G D I R Q A H K(Pam)-NH$_2$ | 4027,69 |

épitopes CTL représentés

| | | | |
|---|---|---|---|
| RPNNNTRKSI | HLA-B27 | T Q G Y F P D W Q N Y | HLA-B62 |
| PPIPVGEIY | HLA-B35 | Y F P D W Q N Y T | HLA-B17, B35 |
| K R W I I L G L N K | HLA-B27 | T P G P G V R Y P L | HLA-B7 |
| L G L N K I V R M Y | HLA-B62 | R Y P L T F G W | HLA-B27.2 |
| Q V P L R P M T Y K | HLA-A3, A11, B27.2 | Y P L T FG W C | HLA-B18 |
| V P L R P M T Y | HLA-B35 | A F H H V A R E L | HLA-B52 |
| A V D L S H F L | HLA-B62 | F L K E K G G L | HLA-B8 |
| A V D L S H F L K | HLA-A11 | | |

L'ensemble des épitopes représentés permettait d'espérer l'induction de réponses CTL chez une importante proportion de la population humaine. à condition de pouvoir bénéficier de l'effet auxiliaire des épitopes HTL non-définis, mais très probablement présents pour de simples raisons statistiques dans l'un quelconque des lipopeptides à condition toutefois de réunir tous les épitopes, et donc, tous les peptides constituants du mélange dans chaque micro-unité de volume.

## 2- Synthèse

L'approche phase solide a été choisie, en utilisant la stratégie Fmoc pour la protection de la fonction a-amine, et *t* Bu pour la protection des chaînes latérales. Le protocole utilisé est un protocole standard basé sur les méthodes de synthèse décrites par **ATHERTON** (Solid-Phase synthesis a practical approach. IRL press, 1989) et **FIELDS et NOBLE** (*Int. J. Pept. Prot. Res.,* 1990, 35, 161-214).

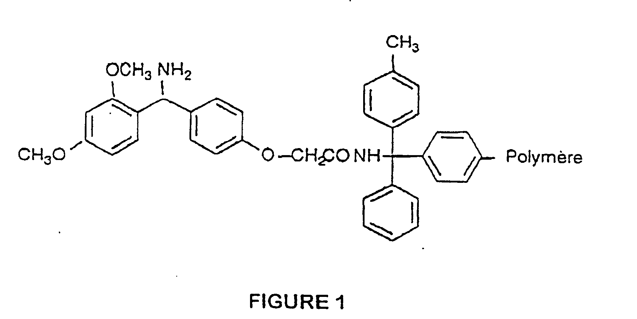

La Fmoc-Lys(Palm)-OH est couplée sur une résine de type KNORR-MBHA (figure 1). Après déprotection de la fonction alpha-amine, on couple le premier acide aminé (Fmoc-Leu-OH dans le cas de NEF 66 par exemple). L'agent de couplage a été le TBTU (3 eq) en présence de DIPEA (4.5 eq), en vérifiant les couplages par un test colorimétrique. Une acétylation systématique a été réalisée après qu'une réaction négative ait été obtenue à ce test, pour minimiser au maximum le risque d'obtenir des peptides à délétion. On répète cette succession d'opérations jusqu'à ce que tous les acides aminés constitutifs de la séquence aient été introduits.

Après la synthèse, et déprotection du groupe Fmoc terminal, les peptides sont déprotégés et clivés par un mélange TFA/eau/DTT (NEF 66, ENV), TFA/eau/DTT/Ac-Trp-OH (GAG 183, GAG 253, NEF 117) ou TFA/eau/EDT/Ac-Trp-OH (NEF 182).

Les peptides sont purifiés chacun sur une colonne Vydac C18 qui a été exclusivement utilisée à cet effet, à température ambiante, avec un système solvant eau-acétonitrile, en tampon perchlorate ou TFA.

Ils sont ensuite mis sous forme d'acétate par échange d'ions sur colonne Dowex SBR, puis lyophilisés dans de l'acide acétique à 40 %.

Chaque peptide provient d'un lot unique de synthèse et purification. Aucune opération de recyclage de fractions de purification n'est pratiquée.

## 3 - Etudes de la solubilité des lipopeptides:

## 3-1) Utilisation de l'eau pure:

Les peptides NEF66, NEF117, NEF182 et ENV ont pu être dissous dans l'eau pure, à des concentrations allant jusque 5 mg/ml. Le peptide NEF117 donne cependant une solution très légèrement opalescente. Les peptides GAG 182 et GAG 253 ne sont pas solubles dans ces conditions.

Le mélange des lipopeptides est cependant soluble dans l'eau pure, indiquant un rôle potentiellement solubilisant des lipopeptides hydrophiles vis-à-vis des peptides peu solubles.

**3-2) Utilisation du DMSO :**

La mise en solution de lipopeptides est fréquemment réalisée à l'aide de solutions aqueuses de DMSO (diméthylsulfoxyde) . Ce solvant organique très puissant est en effet compatible après dilution avec la plupart des essais biologiques réalisés sur cellules ou chez l'animal, voire chez l'homme. L'utilisation de DMSO s'est avérée efficace pour une bonne reprise des peptides GAG 182 et GAG 253; les solutions obtenues ont ensuite pu être diluées par de l'eau pour atteindre une concentration finale de 1 mg/ml dans le DMSO à 20% dans l'eau; dans ces conditions, la plupart des peptides donnent des solutions claires, sauf GAG 183, pour lequel on obtient une suspension.

Il est intéressant de souligner que même dans le cas de solutions claires, et malgré la compatibilité du DMSO avec les filtres Durapore, les solutions de lipopeptides dans le DMSO ne sont pas filtrables sur des filtres de porosité 0,22 $\mu$m, parce qu'elles imposent une pression incompatible avec la résistance mécanique des filtres. Cette observation indique la formation d'agrégats de taille supérieure à 0,22 $\mu$m. Dans certains cas, on a même observé une impossibilité de filtration sur des filtres résistant aux solvants, d'une porosité de 1 $\mu$m, en raison de la formation de gels (cette taille de filtre est en effet utilisée pour filtrer des solutions concentrées de lipopeptides avant purification par RP-HPLC).

**3-3) Utilisation d'acide acétique concentré à 25%:**

La mise en oeuvre de l'indispensable étape de filtration stérilisante nécessite donc l'emploi d'un solvant organique plus apte à dissocier les agrégats, compatible après dilution avec la lyophilisation, et non toxique à faible dose. L'acide acétique a été testé.

Dans un premier temps, une quantité minimale de ce solvant, a été mise en oeuvre qui est définie comme étant la quantité permettant l'obtention de solutions limpides à des concentrations de 5 mg/ml : pour les peptides GAG 183 et GAG 253, 25% d'acide acétique a été introduit; pour les autres peptides, une mise en solution dans l'eau pure a été réalisée.

Les solutions ont été soumises à une analyse par Résonance Magnétique Nucléaire dans un champ de 600 Mhz. Il a été constaté que, malgré l'apparente limpidité des solutions les lipopeptides de cette série. même les lipopeptides les plus hydrophiles, forment des agrégats de taille importante qui interdisent ce type d'étude, en l'absence de signaux résolus.

La mise en solution dans ces conditions ne permet pas la dispersion statistique, au stade moléculaire, de chacun des constituants, malgré l'apparente limpidité des solutions.

**3-4) Utilisation d'acide acétique concentré à 80% :**

L'utilisation d'acide acétique concentré à 80%, a alors été testée. Les peptides ont été mis en solution à une concentration de 1 mM dans 1 ml d'acide acétique à 80% (correspondant à : NEF 66 : 3,86 mg/ml; NEF 117 : 4,02 mg/ml; NEF182 : 3,45 mg/ml;GAG 183 : 3,98 mg/ml; GAG 253 : 4,063 mg/ml: ENV : 4,027 mg/ml).

**Analyse des lipopeptides par RMN du proton à 600 Mhz**

Les échantillons de lipopeptides ont été préparés en dissolvant les lipopeptides dans une solution d'acide acétique (CD3COOD, 99,5% atom D. EURISO-TOP. France)/H20; 80:20 (V:V). 4 $\mu$l d'une solution 50 mM de TMSP [3-(triméthyisilyl)-propane sulfonate de sodium] dans D20 sont ajoutés pour référencer les déplacements chimiques. La concentration finale de chaque peptide est de 1mM dans au moins 2 ml de solvant, qui sont transférés dans des tubes R.M.N. de 8 mm de diamètre (WILMAD 513A-7PP, Interchim, France).

Les spectres de R.M.N. du proton ont été réalisés sur un spectromètre RMN BRUKER DMX600 équipé d'une sonde BBI 8 mm avec gradient z, à une température de l'échantillon à 310°K.

Les expériences en deux dimensions homonucléaires NOESY (Nuclear-Overhauser effect spectroscopy) selon Kumar et al. (1980, Biochem. Biophys. Res; Comm., 95, 1-6) et TOCSY (Total Correlation Spectroscopy) selon Bax et Davis (1985 J. Magn. Reson., 65, 355-360) et Griesinger et al. (1988 J.A.C.S., 110, 7870-7872) ont été obtenues avec 2048 x 512 points complexes et traitées après multiplication dans les deux dimensions par un sinus déplacé de $\pi/4$ avec 2048 x 1024 points, ceci pour une fenêtre spectrale de 12 ppm. Les temps de mélange étaient de 300 ms pour la NOESY et 160 ms pour la TOCSY. Durant le temps de mélange du TOCSY, un MLEV 16 était appliqué avec un champs B1 de 7,8 Khz. Afin d'être dans les mêmes conditions de température, le temps de « spin-lock » du TOCSY était appliqué hors résonance (+ ou - 1 Mhz) dans la NOESY. La suppression de l'eau est achevée en utilisant une légère présaturation de ce signal pendant le temps de relaxation et le temps de mélange de la NOESY.

L'analyse par RMN à haut champ des solutions a montré des signaux parfaitement résolus, permettant des expériences TOCSY-NOESY, et l'attribution séquentielle complète de chaque lipopeptide. Ce résultat signifie la dispersion complète, à l'échelon moléculaire, des lipopeptides dans l'acide acétique à 80%.

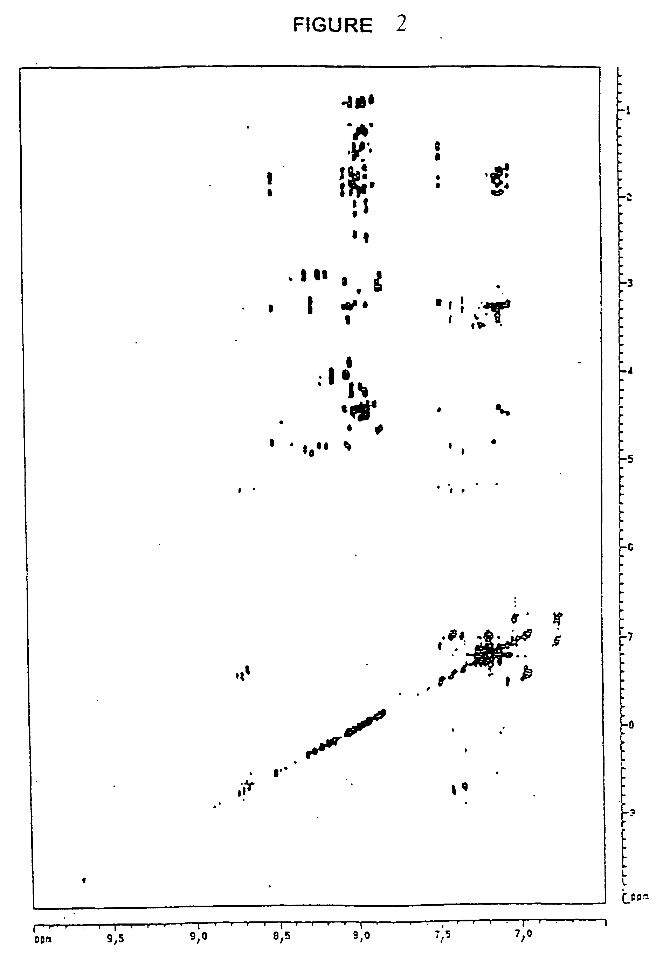

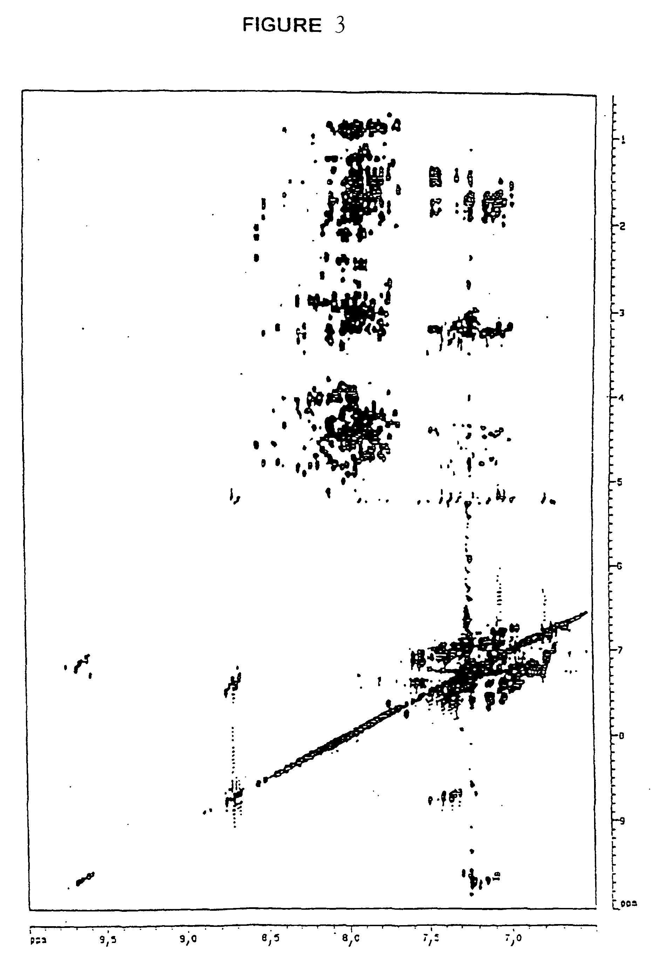

Le spectre RMN 2D du peptide ENV est représenté sur la figure 2 Les spectres de tous les peptides ont pu être obtenus dans les mêmes conditions et interprétés. Afin de vérifier si le mélange des solutions entre-elles n'altérait pas la dispersion des lipopeptides, un spectre RMN 2D du mélange virtuel, a été obtenu par superposition sur une même représentation des 6 spectres obtenus individuellement. Il a été comparé avec le spectre RMN 2D effectivement obtenu par mélange des solutions (figure 3). La résolution des signaux est restée comparable, attestant qu'aucun des peptides n'a altéré la solubilité des autres constituants du mélange.

L'analyse des séquences a nécessité une accumulation des signaux pendant 120 heures pour chaque lipopeptide, période au cours de laquelle aucune altération sensible des peptides n'a pu être détectée, ni par RMN, ni par RP-HPLC. Cette observation a donc permis d'envisager l'utilisation de ce solvant pour la solubilisation des lipopeptides, leur mélange, puis la filtration, même avec un temps de séjour de l'ordre de 1 ou 2 heures, éventuellement nécessaire à la manipulation de volumes relativement importants.

**4 - Etudes de l'étape de filtration stérilisante:**

**4-1) Lipopeptides isolés:**

Des essais de filtration stérilisante ont été réalisés à partir de solutions à 5 mg/ml de chaque lipopeptide dans l'eau pour les peptides NEF66, NEF117, NEF 182 et ENV et dans l'acide acétique à 25% pour les peptides GAG 183 et GAG 253. Les rendements de filtration de 1 ml sur filtres Millex GV SLGV 0130S (0,22 $\mu$m) Millipore, suivie d'une lyophilisation figurent dans le tableau 8 (à la précision du dosage près) .

Ces résultats sont en accord avec les études de solutions réalisées par RMN, et donnent des indications sur la taille des agrégats ou micelles détectés: certains peptides forment des agrégats de taille supérieure à 0,22 $\mu$m, et conduisent par conséquent à des mélanges comportant des microhétérogénéités et une capture simultanée peu probable à l'échelle de la cellule présentatrice d'antigènes.

**4-2) Préparation de divers mélanges de lipopeptides :**

**a) Préparation du lot CK2. Mélange simple des solutions, et obtention d'un mélange limpide, mais microhétérogène:**

Pour la mise en solution des lipopeptides et la préparation du mélange, des solutions d'aspect totalement limpide ont été mélangées entre elles, afin d'évaluer l'éventuelle contribution du caractère tensioactif des lipopeptides ENV, NEF 66, NEF117 et NEF182. Les conditions sont résumées dans le tableau 9.

Les solutions obtenues ont été soumises à l'action d'ultrasons pour faciliter la dispersion des agrégats, mélangées pour donner un volume final de 5,5 ml, le mélange a été de nouveau soumis à l'action d'ultrasons, puis dilué par 9,5 ml d'eau pour obtenir une concentration finale d'environ 8 % en acide acétique (AcOH), compatible avec une lyophilisation de bonne qualité. Cette solution, après un dernier séjour dans le bain à ultrasons, a été filtrée sur filtres Millex GV SLGV 0130S (0,22 $\mu$m) Millipore. Les rendements de filtration des peptides en mélange ont été calculés pour chaque lipopeptide, pour donner les résultats représentés dans le tableau 9 en dernière colonne (à la précision du dosage prés).

L'hétérogénéité des rendements selon les peptides atteste de l'hétérogénéité de la solution. Chaque peptide s'est comporté comme s'il était filtré individuellement : ce comportement est particulièrement évident avec le peptide GAG253, dont le rendement de filtration à partir de cette solution à 8 % d'acide acétique est inférieur au rendement de filtration observé lorsqu'il est filtré seul à partir d'une solution à 25% d'acide acétique. Ce résultat confirme que, en dépit de l'apparente limpidité du mélange en solution acétique diluée, le mélange entre les lipopeptides n'a pas fourni de micro-agrégats ou micelles mixtes, incorporant notamment les peptides les plus hydrophobes. Les échanges de lipopeptides entre micelles se font mal dans ces conditions, et la fonction tensioactive des lipopeptides ENV, NEF66 , NEF 117 et NEF182 ne peut se manifester.

**b) Préparation du lot CK3 : préparation de micelles ou de micro-agrégats mixtes ne comportant pas de microhétérogénéité**

Pour garantir le mélange réel au niveau de chaque micro-unité de volume des différents lipopeptides, une stratégie différente a été suivie :

- chaque lipopeptide a été mis en solution dans l'acide acétique à 80% afin d'exploiter les propriétés dissociantes de ce solvant.
- afin d'exploiter les propriétés de tensioactif cationique attendues des peptides ENV, NEF66, NEF 117 et NEF182 en milieu acide faible lors de l'étape de dilution, les lipopeptides ont été dispersés en solution d'acide acétique à 80% selon la répartition suivante : 1 : ENV. 2 : NEF66, 3 : NEF 117, 4 : NEF182, en terminant par la dispersion des deux lipopeptides les plus hydrophobes dans une solution désormais concentrée en agents dissociants : acide acétique et détergents cationiques . Le cinquième lipopeptide introduit a été GAG 183 et le sixième : GAG 253. Les ultrasons sont utilisés à chaque étape pour assurer une dispersion efficace des agrégats.

Les solutions sont mélangées, puis filtrées sur filtres Millex GV SLGV 0130S (0,22 μm). La filtration nécessite une pression plus faible que lors de la filtration de la solution à 8%. Les récipients utilisés et le filtre sont ensuite rincés avec de l'eau, en quantité suffisante pour obtenir une concentration finale en acide acétique de 8 % (volume final 15 ml comme précédemment), afin d'assurer la qualité de l'étape de lyophilisation. Les rendements de filtration des peptides en mélange ont été calculés pour chaque lipopeptide, pour donner les résultats donnés dans le tableau 10 en dernière colonne (à la précision du dosage près).

L'homogénéité des rendements confirme l'homogénéité de la solution résultant de la dispersion à l'échelon moléculaire au moment de la filtration dans l'acide acétique concentré. La dilution ultérieure ne peut participer à un regroupement de chaque peptide en entités monovalentes, en application des lois de l'entropie. Cette méthode de préparation du mélange a donc permis l'obtention de micelles mixtes, comportant nécessairement chacune une représentation statistique de chaque lipopeptide. Les propriétés tensioactives des lipopeptides peuvent s'exercer et garantir la solubilité dans l'eau des doses de vaccins après lyophilisation ainsi que la stabilité des solutions pendant le temps des manipulations.

### c) Préparation du Lot CK9

La procédure utilisée est la même que pour le lot précédent, aux quantités près. La solution de peptide (20 mg/ml dans l'acide acétique à 80 %) a été filtrée en 4 fois, avec changement de filtre avant leur saturation, en utilisant des membranes identiques (unités stériles STERIVEX GV 0,22 μm Durapore (Millipore), puis complétée avec de l'eau utilisée pour rincer les filtres et diluer. Le volume final atteint était de 1516 ml (dont 154 ml d'acide acétique : solution finale à 10%). Le volume de répartition était de 1,3 ml par dose. Les doses réparties ont été lyophilisées, et dosées en utilisant une méthode de dosage validée par HPLC. Le rendement de filtration pour chaque lipopeptide est indiqué ci-dessous dans le tableau 11, et prend en compte la sensibilité du dosage de chaque lipopeptide.

Lors de la préparation de ce lot, on observe encore une bonne homogénéité des rendements de filtration confirmant la formation de micelles ou micro-agrégats mixtes, chaque micro-unité comprenant une proportion équivalente de chaque constituant du mélange.

Le mélange après dilution et lyophilisation fournit une poudre blanche formant un gâteau compact et homogène, très facilement remis en solution par de l'eau pure, ou un solvant capable de restaurer l'osmolarité de la solution (glucose à 5%, mannitol à 5%). La solution présente une très légère opalescence. Le pH obtenu lors de la reprise par un solvant non tamponné est de 4,90. L'élévation du pH de 1 point provoque la formation lente d'un précipité: ce comportement contribue à la formation d'un dépôt lors de l'injection sous-cutanée ou intramusculaire.

### d) Essai d'uniformité de teneur sur le lot CK9

Selon la Pharmacopée, les poudres pour usage parentéral sont soumises à l'obligation d'uniformité de teneur. L'essai doit être réalisé sur 10 unités prises au hasard, pour lesquelles on réalise individuellement un dosage du principe actif au moyen d'une méthode analytique appropriée. La préparation satisfait à l'essai si la teneur de chaque unité est comprise entre 85 et 115 % de la teneur moyenne.

L'essai a été réalisé sur 15 échantillons pris au hasard, remis en solution et dilués dans de l'acide acétique à 80 % selon une procédure opératoire standardisée, de façon à injecter une proportion toujours identique d'environ 15 μg, définie lors de l'obtention de la droite étalon. Chaque échantillon a été injecté trois fois, le dosage de chaque principe actif correspondant à la moyenne des trois valeurs obtenues.

Les valeurs obtenues figurent dans le tableau 12 ci-après. La répartition des valeurs est clairement statistique. On observe toutes les valeurs à l'intérieur d'un espace défini pour un test bilatéral avec un $P = 0,975$ (sauf trois valeurs aberrantes, toutes retrouvées dans le flacon n°1, pouvant correspondre à une erreur de dilution). Les valeurs minimales et maximales définies se situent à l'intérieur des limites imposées par la Pharmacopée (l'écart observé se situe entre 4 et 14,95% selon les peptides, variation liée à l'imprécision inhérente au dosage).

L'absence de microhétérogénéité de la solution est confirmée par le fait que les doses de vaccins réparties ont satisfait à l'essai d'uniformité de teneur.

**EXEMPLE 2- Préparation d'un mélange de lipopeptides (SIV-Mortara 1) et essai d'immunogénicité chez le macaque.**

**1) Préparation du lot SIV-Mortara 1**

Ce lot de petite taille a été préparé pour réaliser une étude pré-clinique chez le macaque, pour vérifier la tolérance et l'immunogénicité. Ce lot résulte du mélange des lipopeptides suivants:

| nom | formules: |
|---|---|
| NEF 101 | S V R P K V P L R A M T Y K L A I D M S H F I K E K K(Pam)-NH$_2$ |
| NEF 125 | E K G G L E G I Y Y S A R R H R I L D M Y L E K(Pam)-NH$_2$ |
| NEF155 | D W Q D Y T S G P G I R Y P K T F G W L W K L V K(Pam)-NH$_2$ |
| NEF 201 | S K W O D P W G E V L A W K F D P T L A Y T Y E A K(Pam)-NH$_2$ |
| NEF221 | Y T Y E A Y A R Y P E E L E A S Q A C Q R K R L E E G K(Pam)-NH$_2$ |
| GAG165 | K F G A E V V P G F Q A L S E G C T P Y D I N Q M L N C V G D K(Pam)-NH$_2$ |
| GAG246 | Q I Q W M Y R Q Q N P I V G N I Y R R W I Q L G L Q K C V R M Y N P T N K(Pam)-NH$_2$ |
| TT | Ac- Q Y I K A N S K F I G I T E L K K K(Pam)-NH$_2$ |

Leur mélange a été réalisé à partir des solutions dans l'acide acétique concentré, comme lors de la préparation du lot CK3.

**2) Immunogénicité chez le macaque.**

**a) Matériels et méthodes**

Les macaques, numérotés respectivement 102, 105. 109, 117, 120, 125, 127 et 129 ont été immunisés par injection sous-cutanée du lot préparé ci-dessus (500 μg), dans de l'eau stérile et ont été réinjectés dans un délai de trente jours et de soixante jours.
Ces immunisations ont été effectuées en accord avec les directives de l'Union Européenne.

**Obtention de lignées CTL**

Des cellules sanguines (PBMC) ont été isolées par centrifugation dans un gradient de densité à travers un milieu de séparation de lymphocytes (Pharmacia, Uppsala, Suède). Elles ont été utilisées immédiatement, ou stockées à -180°C dans de l'azote liquide. Des lignées CTL anti-peptide ont été obtenues en cultivant les PBMC des singes (2 x 10$^6$ cellules/ml) dans des plaques de microtitration, dans du RPMI 1640 complété avec de la pénicilline (100U/ml), de la streptomycine (100 μg/ml) de la L-glutamine (2 mM), des acides aminés non essentiels (1%), du pyruvate de sodium (1 mM), du tampon HEPES (10 mM), du 2-mercaptoéthanol (2 x 10$^{-5}$ M) et du sérum de veau foetal inactivé par la chaleur (FCS) à10%.
Le mélange des sept peptides libres, c'est-à-dire sans motif lipidique. (5 μm de chaque) correspondant aux séquences lipopeptidiques a été ajouté dans chaque puits.
Les plaques ont alors été incubées durant trois jours à 37°C et de l'Interieukine-2 a été ajouté dans chaque plaque (10 IU/ml).
Après sept jours et quatorze jours, les cellules effectrices ont été stimulées en ajoutant de nouveaux PBMC autologues, qui ont été mis en contact durant deux heures avec le mélange de peptide (5 μm de chaque), puis lavés et irradiés (4000 rads).

**Mise en évidence de la prolifération des cellules T**

Des cellules PBMC (2 x 10$^5$ dans 200 μl par puits) ont été cultivées dans des plaques contenant 1 μg/ml du lipopeptide TT (830-846), et 10μg/ml du peptide issu de la toxine tétanique (TT).
Après cinq jours de culture, 1 μCi de [$^3$H]TdR a été ajouté dans chaque puits et l'incubation a été poursuivie durant dix huit heures. Les cellules ont été alors récoltées à l'aide d'un récolteur automatique de cellules puis l'incorporation de la thymidine tritiée a été quantifiée à l'aide d'un compteur à scintillation.

**Analyse phénotypigue des lignées cellulaires CTL**

Le phénotype des lignées cellulaires a été déterminé le jour où le test de largage du chrome a été effectué, en incubant les cellules avec des anti-CD4 conjugués à du FITC (OKT4, Ortho Diagnostic Systems, Raritan, NJ) et avec un anti-CD8 conjugué à de la phycoérythrine (Leu-2a, Becton Dickinson, Mountain View, CA) durant trente minutes à 4°C. Les cellules ont été lavées avec du tampon PBS puis le pourcentage de cellules colorées a été déterminé à l'aide d'un cytomètre de flux Epics Elite (Coulter, Margency, France). Des anticorps présentant un mélange d'isotypes ont été utilisés comme témoins.

**Transformation in vitro des lignées cellulaires B (B-LCL)**

Les lignées cellulaires B (B-LCL) ont été obtenues en incubant des dilutions en séries de PBMC à l'aide du surnageant de la lignée cellulaire S 594. Cette lignée est infectée par le virus de l'herpès du babouin qui immortalise les cellules (herpès virus papio). Les B-LCL ont été ensuite cultivées dans du milieu de culture complémenté avec 10% de FCS.

**Test de relarguage du chrome.**

Des cellules cibles ont été sensibilisées avec les peptides. $10^6$ cellules B-LCL ont été incubées soit durant la nuit soit durant 1 heure, respectivement avec des peptides longs ou courts (gamme de concentration $10^{-5}$ M-$10^8$M) à 37°C dans une atmosphère humidifiée à 5% de $CO_2$. Afin d'obtenir les cellules cibles présentant les produits du gène SIVmac, les B-LCL ont été incubés à une concentration de $10^6$ cellules/ml avec un virus de la vaccine recombinante (20 PFU/cellule) durant dix huit heures dans les mêmes conditions. Les BLCL ont été ensuite lavées et marquées avec $100\mu$m Ci $Na_2{}^{51}CrO_4$ (NEN Life Science Products, Courtaboeuf Les Ullis, France) durant 1 heure, lavées deux fois et utilisées comme cellules cibles. Le relarguage du $^{S1}Cr$ a été effectué dans des plaques de microtitration. L'activité cytolytique des lignées cellules anti-SIV a été mesurée en mélangeant $5 \times 10^3$ cellules cibles marquées au chrome avec des cellules effectrices, dans des rapports cellules effectrices/cellules cibles divers, dans un volume final de 200 $\mu$l/ puits. Les plaques ont été incubées durant 4 heures à 37°C, puis 100 $\mu$l de surnageant ont été pris dans chaque puits et analysés dans un compteur de rayonnement gamma.

Le relarguage spontané du chrome a été déterminé en incubant les cellules cibles avec du milieu seul. Il n'excède jamais 20% du chrome total incorporé.

Le relarguage spécifique du chrome a été mesuré de la manière suivante:

$$100 \ x \ (cpm \ expérimentaux - cpm \ spontané)/(cpm \ maximum-cpm \ spontané).$$

La variation à l'intérieur d'un échantillon n'excède jamais plus de 5%.

**b) Résultats**

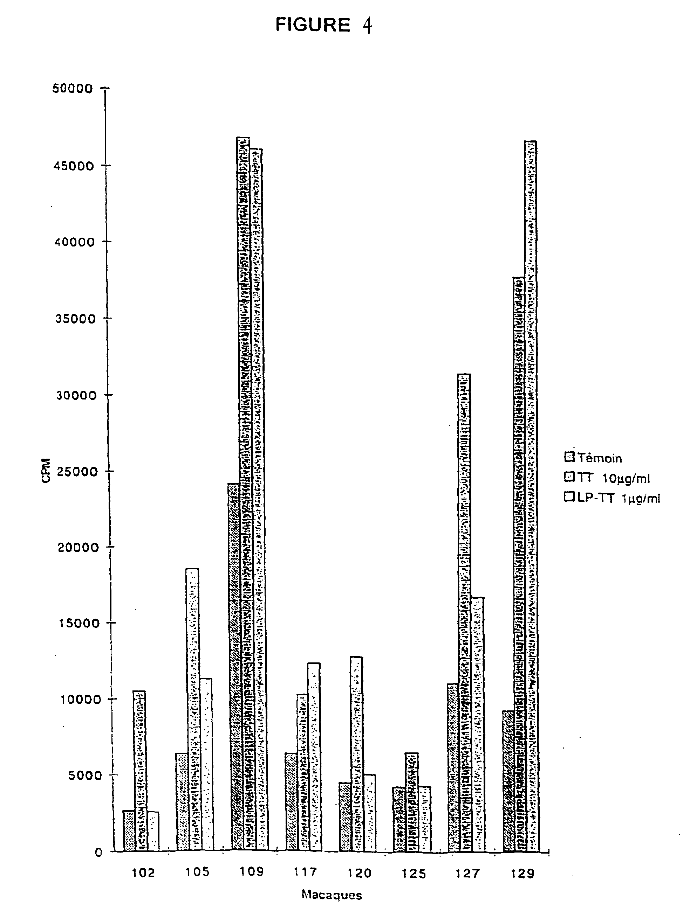

La figure 4 illustre la réponse T auxiliaire des huit macaques.

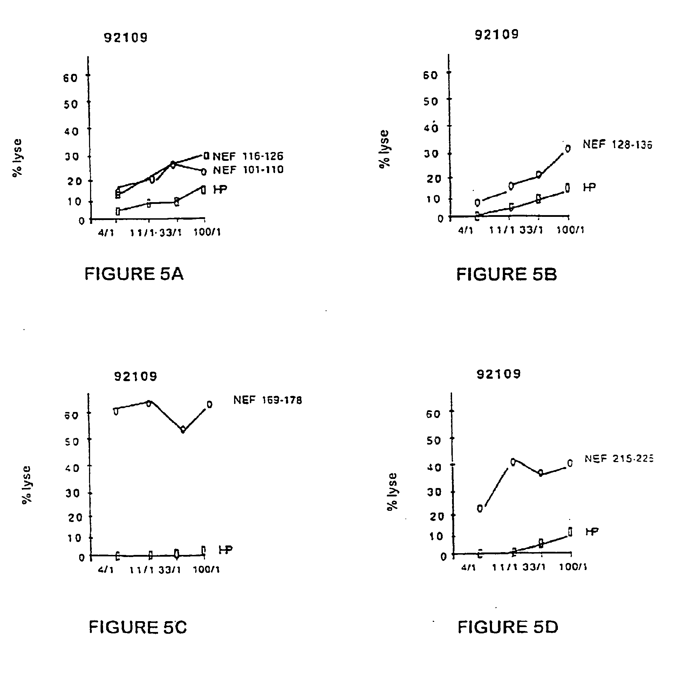

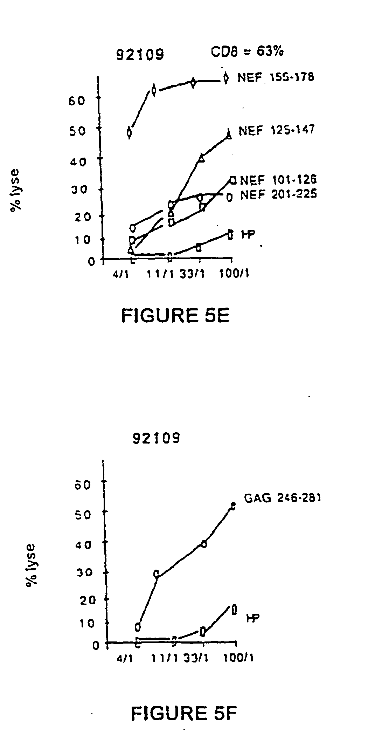

Les figures 5 a 11 illustrent la réponse cytotoxique des macaques.

Les résultats des immunisations avec divers peptides sont résumés dans le tableau 13.

Ils montrent que sept des huit macaques testés reconnaissent divers peptides, les macaques n°109, 129 et 127 montrant une réponse particulièrement importante.

L'efficacité de l'induction d'une réponse CTL confirme que les CPA des animaux ont pu capturer et présenter un ou plusieurs épitopes CTL,et simultanément l'épitope auxiliaire fort présent dans l'anatoxine tétanique et reconnu par certains des animaux.

**EXEMPLE 3:**

**Préparation d'un mélange de lipopeptides (Lot HG 1) en vue d'un essai clinique chez l'homme.**

Un mélange de lipopeptides a été défini en vue de la réalisation d'un essai clinique (VAC10), associant au sein de micelles le même peptide TT à des séquences choisies sur le principe de sélection développé pour VAC04 (existence d'un ou plusieurs épitopes CTL par séquence).

- la cystéine du peptide NEF117 a été remplacée par une Leucine : après synthèse et essai sur test cellulaire de plusieurs analogues du nonapeptide épitope CTL renfermant cet acide aminé, il a en effet été observé que ce remplacement était possible; cette modification permet d'éviter les problèmes de stabilité dus à la formation de pont disulfure.
- le peptide GAG 17 a été choisi parmi d'autres candidats pour son caractère nettement tensioactif cationique susceptible d'aider au maintien en solution des autres peptides, et en particulier de GAG253. toujours présent dans le mélange, en raison de son immunogénicité chez l'homme.

La composition de ce mélange, dans laquelle Pam représente un motif dérivé de l'acide palmitoïque et Ac le groupe acétyl, est la suivante:

GAG 17 EKIRLRPGGKKKYKLKHIV K(Pam)-NH2
GAG 253 NPPIPVGEIYKRWIILGLNKIVRMYSPTSILD K(Pam)-NH2
POL 325 AIFQSSMTKILEPFRKQNPDIVIYQYMDDLY K(Pam)-NH2
NEF 66 VGFPVTPQVPLRPMTYKAAVDLSHFLKEKGGL K(Pam)-NH2
NEF 116 HTQGYFPDWQNYTPGPGVRYPLTFGWLYKL K(Pam)-NH2
TT Ac-CIYIKANSKFIGITELKK K(Pam)-NH2

L'ensemble de ces peptides a été synthétisé comme indiqué dans les exemples précédents. Le mélange des solutions a été réalisé sur un échantillon de 5 mg de chaque peptide, dissous à une concentration de 20 mg/ml dans l'acide acétique à 80% puis mélangé dans l'ordre suivant : 1 : GAG17; 2 : NEF66; 3 : NEF 116; 4 : TT ; 5 : GAG253 ; 6 : POL 325.

Les rendements de l'opération de filtration des solutions acide acétique concentrées, suivie d'une dilution par l'eau se sont avérés comparables aux rendements observés pour les mêmes opérations avec le mélange CK3 (à la précision du dosage près). L'homogénéité des solubilités et de comportements vis-à-vis de la filtration stérilisante en dépit des hétérogénéités de comportement chimique au stade individuel traduit la formation de micelles mixtes.

**EXEMPLE 4:**

**Préparation d'un mélange de lipopeptides dérivés de l'antigène LSA3 en vue d'un essai pré-clinique de vaccination contre le stade intrahépatique de *Plasmodium falciparum,* réalisé chez la souris et le chimpanzé, puis d'un essai clinique chez l'homme.**

LSAE CT1    LLSNIEEPKENIIDNLLNNIK(Pam)-NH$_2$
LSA3    NRI Ac-DELFNELLNSVDVNGEVKENILEESQK(Pam)-NH$_2$
LSA3    NRII Ac-LEESOVNDDIFNSLVKSVQQEQQHNVK(Pam)-NH$_2$
LSA3    RE K(Pam)VESVAPSVEESVAPSVEESVAENVEESVAENV-NH$_2$

L'ensemble de ces peptides a été synthétisé comme indiqué dans l'exemple 1. Le mélange des solutions a été réalisé sur un échantillon de 5 mg de chaque peptide préalablement dissous à une concentration de 20 mg/mL dans de l'acide acétique à 80% puis mélangés dans l'ordre suivant : 1 LSA3 NRI; 2:LSA3 NRII; 3:LSA3 CT1; 4: LSA3 RE. Les rendements de l'opération de filtration des solutions d'acide acétique concentrées, suivie d'une dilution par l'eau, se sont avérés comparables pour tous les lipopeptides.

**EXEMPLE 5:**

**Etude de la réponse immunitaire chez l'homme après injection de micelles du lot CK9.**

**1. Matériels et méthodes**

**Micelles utilisées:**

Les micelles qui ont été injectées ont été obtenues tel que décrit dans l'exemple 1 pour le lot CK9.

**Peptides longs et peptides courts.**

Les peptides longs suivants correspondant aux lipopeptides immunogènes ont été synthétisés (les positions des acodes aminés sur les protéines NEF, GAG et ENV sont indiquées entre parenthèses) : N1 (NEF 66 à 97), N2 (NEF 117 à 147), N3 (NEF 182 à 205), G1 (GAG 183 à 214), G2(GAG 253-284) et E (ENV 303 à 335).

Les peptides courts suivants recouvrant les séquences des lipopeptides, déjà connus pour être des épitopes CTL minimaux, ont été synthétisés par Neosystem (Strasbourg, France):

NEF 121-128, NEF 137-145, NEF 184-191 et NEF 195-202 restreints à HLA-A1.
NEF 136-145, NEF 190-198 et GAG 183-191 restreints à HLA-A2.
NEF 73-82, NEF 84-92 et EBN 416-424 HLA restreints à HLA-A11.
NEF 90-97 et NEF 182-189 restreints à HLA-B8.
NEF 134-141 et GAG 263-272 restreints à HLA-B27.
NEF 135-143 restreints à HLA-B18.

**Protocole d'immunisation :**

Des volontaires ont été immunisés par injection souscutanée des micelles, ou des six lipopeptides correspondant, en présence d'adjuvant QS21. Les lipopeptides ou les micelles ont été injectés de manière différente en fonction des individus.

Les volontaires V4.6, V4.15, V4.16, V4.17, V4.18 et V4.28 ont été immunisés avec les six lipopeptides sous forme de micelles.

Le volontaire désigné V4.6 a reçu 250 $\mu$g de chacun des lipopeptides.

Les volontaires désignés V4.15, V4.16, V4.17, V4.18 et V4.28 ont été immunisés avec 500 $\mu$g de chacun des six lipopeptides.

Les volontaires V4.5, V4.1, V4.19, V4.21, V4.32 et V4.34 ont été immunisés avec les six lipopeptides en présence d'adjuvant QS21.

Le volontaire V4.5 a reçu 100 $\mu$g des 6 lipopeptides, tandis que les volontaires V4.1, V4.19,V4.21, V4.32 et V4.34 ont reçu chacun 500$\mu$g des six lipopeptides.

Tous les volontaires ont été immunisés trois fois avec le mélange des six lipopeptides, les deux dernières injections étant effectuées 4 semaines et 16 semaines respectivement après la première injection.

Des échantillons de sang ont été collectés après la première injection (appelé ci-après semaine 0) et 20 semaines après la première injection (semaine 20).

Les cellules mononucléaires du sang périphérique (PBMC) et le sérum ont été isolés par des méthodes classiques, et ont été congelés.

**Détection par ELISA des anticorps anti-peptides du VIH, de type immunoglobuline G (IgG).**

Des puits de plaques de polystyrène ont été recouverts avec 5 $\mu$g/ml des peptides (N1, N2, N3, G1, G2 ou E) durant une nuit à 4°C. La saturation a été effectuée à l'aide d'une solution de PBS contenant 0,1% de Tween 20 et 3% de sérum albumine bovine (BSA). Des sérums dilués (1/100) ont été incubés dans les puits recouverts durant une nuit à 4°C et les anticorps liés ont été détectés à l'aide d'IgG de chèvre anti-humain conjuguées à de la phosphatase alcaline (1/5000, Sigma). L'activité phosphatase a été mesurée en utilisant le 4-méthyl-umbelliferyl phosphate comme substrat (Sigma), et la mesure de la fluorescence a été effectuée à 360/460 nm dans un Cytofluor 2300 (Millipore).

**Mesure des réponses cellulaires T dirigées à l'encontre de peptides du VIH.**

Des PBMC ($10^5$ par puits) ont été cultivés dans un milieu complet avec 1 $\mu$g/ml ou 0,2 $\mu$g/ml de peptides solubles (N1, N2, N3, G1, G2 ou E). La prolifération a été déterminée après 5 jours de culture en ajoutant 1 $\mu$Ci/puits de thymidine tritiée (NEN, Paris) 12 heures avant leur récolte.

La capacité des PBMC à proliférer in vitro a été vérifiée à l'aide de cultures indépendantes effectuées durant 5 jours avec de la phytohémaglutinine A (PHA) du PPD (Tuberculin purified derivate Référence Statens Serumins Institute n°2390) de la toxine tétanique (TT) et du SEB (Entérotoxine B du staphylocoque doré, Référence SIGMA S4881), respectivement à 1 $\mu$g/ml et 10$\mu$g/ml.

L'élimination des cellules T CD4$^+$ et CD8$^+$ des PBMC a été effectuée à l'aide d'immunoglobulines anti-souris et par activation du complément. En résumé $10^7$ PBMC ont été incubés dans 1 ml de milieu dépourvu de sérum albumine bovine durant 30 minutes à 4°C avec 2 $\mu$g d'anticorps monoclonaux OKT4 ou OKT8 (Orthodiagnostic Systems).

1 ml de complément de sérum de lapin dilué (Hoechst Behring, RUEIL, FRANCE) a été ajouté durant 45 minutes à 37°C. La suspension cellulaire a été lavée deux fois afin d'éliminer le complément non lié. Les cellules resuspendues ont été analysées en utilisant la cytométrie de flux. L'analyse des phénotypes à l'aide d'anticorps anti-CD4[+] et anti-CD8[+] a été effectuée afin de vérifier l'enrichissement. Finalement les cellules résultant de l'élimination des cellules CD4[+] et CD8[+] ont été testées dans un test de prolifération.

### Obtention de lignées cellulaires CTL

La stimulation in vitro des PBMC a été effectuée en mélangeant $10^6$ PBMC (cellules répondantes) avec $10^6$ cellules stimulantes irradiées (PBMC autologues incubées pendant 2 heures avec différents peptides) dans du milieu de culture RPMI complet (RPMI 1640 complété avec 100 U/ml de pénicilline, 100 $\mu$g/ml de streptomycine, L-glutamine 2mM, pyruvate de sodium 1 mM, Hepes 10 mM , des acides aminés non essentiels et 10% de sérum albumine bovine inactivée par la chaleur).

10 U/ml d'Interleukine-2 ont été ajoutés après 3 jours. Les cellules répondantes ont été restimulées chaque semaine durant 3 ou 4 semaines à l'aide de peptides incubés avec des PBMC autologues (préparés de la même manière qu'au jour 0), dans un milieu complémenté avec 10 U/ml d'Interleukine-2. Après 3 ou 4 stimulations, les cellules CTL ont été testées en utilisant comme cible la lignée cellulaire autologue EBV incubée durant la nuit avec 10 $\mu$g des différents peptides (N1, N2, N3, G1, G2 ou E) pour $10^6$ cellules.

Pour obtenir des cellules cibles présentant les produits du gène HIV, des cellules cibles EBV ont été infectées, à raison de $10^6$ cellules/ml avec un virus de la vaccine de type sauvage (WT) ou avec des virus de la vaccine recombinants HIV-1/LAI, HIV-1/MN, HIV/A ou HIV/ROD NEF, durant une nuit (20PFU/cellules).

Les différentes cellules cibles ont été ensuite lavées et marquées avec 100 $\mu$Ci de $Na_2$ $^{51}CrO_4$ (NEN Life Science Products, Les Ullis, France).

L'activité cytolytique a été mesurée dans un test de relarguage du $^{51}Cr$, durant 4 heures. Le relarguage spontané moyen n'excède pas 20% de l'incorporation totale de $^{51}Cr$.

Les résultats sont exprimés de la manière suivante:

$$\text{relarguage spécifique du chrome} = 100 \times (\text{nombre de coups mesurés /nombre de coups spontanés par minute})/(\text{nombre maximum de coups par minute - nombre de coups spontanés par minute}).$$

L'élimination des cellules CD4[+] ou CD8[+] à partir des PBMC a été effectuée comme décrit ci-dessus.

### Test ELISPOT Interféron-$\gamma$

Des plaques de nitrocellule de 96 puits (MultiScreen-HA, Millipore S.A., Molsheim, France) ont été recouvertes avec 5 $\mu$g/ml d'anticorps de souris anti-Interféron-$\gamma$ humain, en tant qu'anticorps de capture (Genzyme Corporation, Cambridge, Massachusetts , USA) durant une nuit à 4°C.

Après lavage, les puits ont été saturés avec du milieu complet RMPI et des PBMC récemment isolées, ou conservées au froid, ont été ajoutées (2 x $10^5$ cellules par puits) avec différents peptides correspondant à des épitopes minimaux CD8[+] (10 $\mu$g/ml).

Après 24 heures d'incubation à 37°C dans un incubateur (5% de $CO_2$), les plaques ont été lavées et incubées durant 2 heures avec 100 $\mu$l d'anticorps polyclonaux de lapin anti-inféron-$\gamma$ humain (1/250, Genzyme). Après lavage, un conjugué anti-IgG de lapin-biotine (1/500, Boehringer Mannheim France S.A., Meylan, France) a été incubé durant 1 heure. Finalement, de l'extravidine marquée par de la phosphatase alcaline (Sigma-Aldrich Chimie S.A.R.L. St-Quentin Fallavier, France) a été ajoutée durant 1 heure.

100 $\mu$l de substrat chromogénique de la phosphatase alcaline (Bio Rad Laboratories, Hercules, CA, USA) ont été ajoutés pour développer les tâches. Les tâches bleues ont ensuite été comptées en utilisant un microscope

Le témoin négatif consiste en des PBMC incubées seules dans le milieu, ou incubées avec un peptide correspondant à un épitope CD8[+] dérivé du virus VIH présenté par des HLA adaptés.

Le témoin positif consiste à activer des PBMC avec 50 mg/ml de PMA (Phorbol myristate acétate, référence SIGMA P 8139) et 500 ng/ml d'ionomycine (100 à 300 PBMC par puits ont été ajoutées).

Cette forte stimulation mitogène permet de mesurer la viabilité des lymphocytes T, et de vérifier ainsi la qualité de leur conservation au froid.

## 2. Résultats

### Tolérance du traitement

Les effets secondaires résultant de l'injection des lipopeptides ont été peu conséquents. On a observé une réaction épidermique à l'endroit de l'injection. Les réactions locales consistent en de petits érythèmes qui ne durent plus de 24 à 48 heures. En aucun cas ces effets n'ont été associés avec des symptômes systémiques. Ces observations indiquent que les lipopeptides sont bien tolérés chez les individus normaux.

### Induction spécifique d'une réponse humorale à l'encontre des peptides du VIH-1

Des échantillons de sérum ont été collectés avant le début de la vaccination (semaine 0) et à la vingtième semaine, après la troisième injection.

Les sérums des volontaires immunisés ont été testés par ELISA pour la présence d'anticorps IgG dirigés à l'encontre des peptides de NEF (N1, N2, N3), GAG (G1, G2) et d'ENV (E).

Aucune IgG spécifique des peptides du VIH n'a été détectée avant l'injection chez les douze sujets présentés dans le tableau 14.

A la vingtième semaine, des anticorps IgG anti-N1 ont été détectés seulement dans cinq des sujets vaccinés (V4.6, V4.28, V4.1 (SQ21), V4.32(QS21) et V.4.34 (QS21)), et des anticorps IgG anti-N2 ont été détectés dans les sérums de dix des sujets, parmi les douze vaccinés. Aucun anticorps de type IgG anti-N3 n'a été détecté. Le titre en anticorps anti-N2 est négatif dans les sérums des individus V4.17 et V4.18. Le titre en anticorps est trois à cinq fois supérieur au témoin négatif dans les sérums de V4.15, V4.16, V4.1 (QS21) V4.5 (QS21) et V4.21(QS21). Le titre en anticorps est de 5 à 10 fois supérieur à celui du témoin négatif pour les sérums des individus V4.6, V4.19(QS21) et V4.28. Finalement, les sérums des patients V4.32(QS21) et V4.34 (QS21) présentent un titre en anticorps au moins 10 fois supérieur à celui du témoin négatif.

Après 3 injections aucun anticorps IgG anti-G1 n'a été détecté, mais les anticorps IgG anti-G2 ont été détectés dans les sérums des 12 individus vaccinés. Le titre en anticorps anti-G2 est 2 à 3 fois supérieur au témoin négatif pour le patient V4.18(QS21), le titre en anticorps est 5 à 10 fois supérieur au témoin négatif pour les individus V4.16, V4.17, V4.5(QS21), et V4.19(QS21) et V4.21(QS21). Les sérums des patients V4.6, V4.15, V4.28, V4.1 (QS21), V4.32(QS21) et V4.34(QS21) ont un titre en anticorps plus de dix fois supérieur au témoin négatif. Les sérums de 6 des 12 individus testés, V4.28, V4.1 (QS21), V4.5(QS21), V4.19(QS21), V4.32(QS21) et V4.34(QS21) contiennent des anticorps anti-E spécifiques.

### Réponse T cellulaire auxiliaire spécifique des peptides du virus VIH-1.

Les réponses prolifératives vis-à-vis des peptides solubles obtenus avec les cellules PBMC des différents individus vaccinés apparaissent sur le tableau 15.

Les peptides NEF, GAG et ENV entraînent une prolifération chez les PBMC de donneurs seulement, après la vaccination. Les PBMC d'individus immunisés avec les lipopeptides (avec ou sans l'adjuvant QS21) prolifèrent au moins à l'encontre d'un peptide après 20 semaines (4 semaines après la troisième injection, pour 8 sujets sur 10 présentés dans le tableau 15).

Aucune prolifération n'est observée pour les PBMC des individus V4.15 et V4.17.

Les PBMC de l'individu V4.6 prolifèrent en réponse aux peptides N3, G1 et G2 avec un index de prolifération compris entre 4 et 10. Une induction de la prolifération en réponse à G1, G2 et E est observée avec les PBMC de l'individu V4.16 Des PBMC de l'individu V4.28 sont capables de proliférer en réponse aux peptides N1, N3, G2 et E. Une réponse proliférative, à l'encontre des peptides N1, G2 et E est observée pour les PBMC de l'individu V4.5 vacciné.

Une forte prolifération est observée en réponse aux peptides N1, G2 et E avec les PBMC de l'individu V4.19 (QS21), qui sont par ailleurs aussi capables de proliférer en présence de N2 et N3.

Les PBMC de l'individu V4.21 prolifèrent en présence de N1 et de G2, tandis que ceux de l'individu V4.32 prolifèrent seulement en présence de G2.

Une réponse proliférative est observée en présence de N1, N3, G2 et E avec les PBMC de l'individu V4.34 (QS21).

De manière globale la troisième immunisation avec les lipopeptides induit une réponse proliférative à l'encontre du peptide N1 pour cinq des dix sujets traités, à l'encontre de N2 pour un des dix sujets traités, N3 pour quatre des dix sujets traités, G1 pour deux des dix sujets traités, G2 pour huit des dix sujets traités et finalement E pour des cinq des dix sujets traités.

Les expériences d'élimination effectuées avec les PBMC de différents individus vaccinés montrent que la prolifération des PBMC récupérés après vingt semaines se fait préférentiellement par l'intermédiaire des cellules T CD4$^+$

auxiliaires.

### Induction de l'activité CTL spécifique du VIH

Les PBMC obtenues avant et après les immunisations ont été stimulées in vitro et testées pour leur activité CTL spécifique du VIH.

Les résultats d'expérimentations représentatives sont présentés dans le tableau 16.

L'activité CTL spécifique a été testée à l'encontre de la lignée cellulaire EBV autologue, incubée avec ou sans les peptides NEF, GAG, ENV. Aucune réponse anti-HIV a été détecté avec les PBMC récupérées avant l'immunisation. Une activité spécifique CTL a été détecté chez les PBMC collectées trois semaines après l'immunisation chez neuf des douze individus.

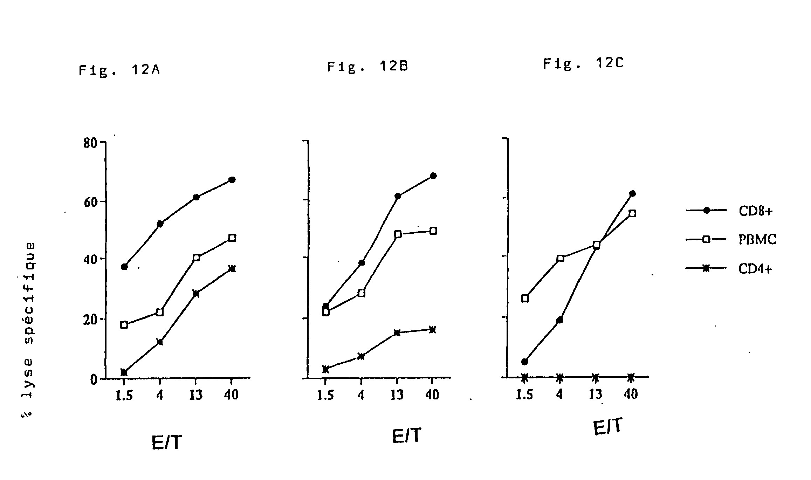

Le tableau 16 résume l'activité cytotoxique de huit des individus vaccinés, l'activité d'un autre de ces individus étant représentée sur la figure 12.

Au moins un peptide contenu dans le vaccin lipopeptidique induit des cellules effectrices CTL spécifiques reconnaissant des peptides HIV.

Par exemple les PBMC de l'individu V4.6 reconnaissent dans un test cytotoxique les cellules EBV autologues impulsées avec les peptides G2 et E . Les PBMC de l'individu V4.16 reconnaissent le peptide N3 et E. Le pourcentage de lyse est variable, faible pour l'individu V4.16 reconnaissant le peptide N3, intermédiaire pour l'individu V4.18 avec le peptide N1 et fort pour l'individu V4.5(QS21) avec les peptides N2 et G2.

On notera qu'une activité CTL spécifique était aussi générée à l'encontre de peptides contenant un épitope minimal CD8+ HIV (individus V4.16 et V4.28).

Afin d'évaluer si les cellules effectrices sont des cellules T CD8+, comme on peut s'y attendre pour des CTL spécifiques d'antigènes restreints de classe 1, les lymphocytes T CD8+ ou CD4+ ont été éliminés des PBMC et on a effectué un test de cytotoxité.

Dans les expériences représentatives effectuées avec des PBMC de l'individu V4.1 (figure 12), on a observé une lyse efficace des cellules EBV autologues incubées avec des peptides du VIH, pour les PBMC et les cellules CD8+. L'enrichissement en cellules CD8+ permet d'augmenter le pourcentage de lyse spécifique. Ces résultats confirment que l'activité cytotoxique anti-HIV passe par l'intermédiaire des cellules T CD8+.

Il était aussi important de déterminer si ces cellules T cytotoxiques (CTL) reconnaissent et lysent des cellules infectées par des virus.

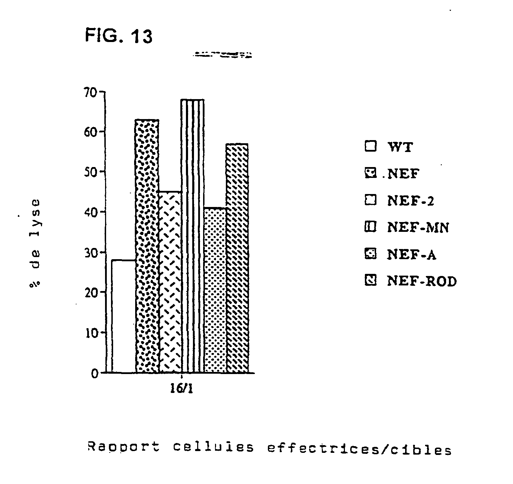

Aussi des PBMC de l'individu V4.5 collectées 20 semaines après l'immunisation, et stimulées in vitro avec le peptide N2 ont été testées pour leur activité CTL à l'encontre de cibles autologues infectées avec différents virus exprimant la protéine NEF recombinante. Les résultats d'expériences représentatives sont présentés dans la figure 13. Des CTL anti-peptide N2 obtenues à partir de l'individu V4.5 (QS21) reconnaissent un antigène naturellement modifié par des EBV-LCL autologues infectées par des virus recombinants de la vaccine codant des gènes HIV-NEF obtenus à partir de différentes souches de HIV.

Pour le même rapport effecteur/cible, les CTL spécifiques du virus HIV reconnaissent NEF-LAI et NEF-MN avec la même efficacité. Un pourcentage plus faible de lyse spécifique est obtenu pour la protéine NEF-A ou la protéine NEF-ROD.

Ces résultats indiquent que les CTL obtenues après vaccination par des lipopeptides sont capables de reconnaître différentes souches du virus VIH.

### Cellules T CD8± sécrétant ex-vivo de l'interféron-γ, spécifiques du virus VIH.

Les cellules effectrices T CD8+ peuvent exercer une activité lytique et/ou produire des lymphokines. Aussi la quantité de cellules T CD8+ sécrétant de l'Interféron-γ a été évaluée par un test ELISPOT spécifique.

Une étude récente a montré que l'inactivation intracellulaire du virus de l'hépatite B se fait par l'intermédiaire de cellules T CD8+ sécrétant de l'Interféron-γ spécifiques et cytotoxiques, qui induisent une immunité protectrice. Cette approche permet d'identifier des épitopes de cellules T CD8+ minimaux par sensibilisation de PBMC avec des peptides courts. Tous les peptides courts utilisés sont déjà décrits comme étant des épitopes CTL (voir plus haut).

On a aussi quantifié par un ELISPOT ex vivo le nombre de cellules T CD8+ sécrétant de l'Interféron-γ spécifique des peptides du VIH dans des PBMC de sujets vaccinés (tableau 17).

### Conclusion

Cette étude a permis de montrer qu'un vaccin lipopeptidique sous forme de micelles, et dépourvu d'adjuvant, contenant différents épitopes VIH, contenu dans les protéines virales NEF, GAG et ENV du virus VIH, est capable d'induire une forte et persistante réponse multiépitopique B et T chez l'homme.

**TABLEAU 1: Epitopes de BCR-ABL**

| Peptide | Séquence | Fixation au HLA |
|---|---|---|
| 247-255 | EDAELNPRF | B44 |
| 488-496 | SELDLEKGL | B44 |
| 768-776 | DELEAVPNI | B44 |
| 901-934 b2a2 | KEDALQRPV | B44 |
| 902-935 b2a2 | EDALQRPVA | B44 |
| 986-994 | GEKLRVLGY | B44 |
| 1176-1184 | EDTMEVEEF | B44 |
| 1252-1260 | MEYLEKKNF | B44 |
| 1691-1699 | NEEAADEVF | B44 |
| 49-57 | VNQERFRMI | B8 |
| 580-588 | LFQKLASQL | B8 |
| 722-730 | ARKLRHVFL | B8 |
| 786-794 | ALYLKISQI | B8 |
| 886-893 | CVKLQTVH | B8 |
| 928-936 b3a2 | KALQRPVAS | B8 |
| 1830-1838 | GAKTKATSL | B8 |
| 1975-1983 | IQQMRNKFA | B8 |
| 1977-1984 | QMRNKFAF | B8 |
| 252-260 | NPRFLKDNL | B7 |
| 329-338 | TPDCSSNENL | B7 |
| 693-701 | TPRRQSMTV | B7 |
| 1058-1066 | SPGQRSISL | B7 |
| 1196-1205 | HPNLVQLLGV | B7 |
| 1560-1569 | SPKPSNGAGV | B7 |
| 1717-1725 | KFLRRQVTV | B7 |
| 1878-1884 | SPAPVPSTL | B7 |
| 36-44 | ERCKASIRR | B27 |
| 71-79 | DRQRWGFFRR | B27 |
| 575-583 | QRVGDLFQK | B27 |
| 834-842 | FRVHSRNGK | B27 |
| 642-650 | LLYKPVDRV | A2 |
| 684-692 | FLSSINEEI | A2 |
| 708-716 | QLLKDSFMV | A2 |
| 714-722 | FMVELVEGA | A2 |
| 817-825 | KLSEQESLL | A2 |
| 881-889 | MLTNSCVKL | A2 |
| 908-917 | GLYGFLNVIV | A2 |
| 912-920 | FLNVTVHSA | A2 |
| 1240-1248 | VLLYMATQI | A2 |
| 1903-191 | FIPLISTRV | A2 |
| 1932-1940 | VVLDSTEAL | A2 |
| 50-58 | NQERFRMIY | A1 |
| 223-231 | VGDASRPPY | A1 |
| 549-558 | KVPELYEIHK | A3/A11 |
| 583-591 | KLASQLGVY | A3/A11 |
| 715-724 | MVELVEGARK | A3/A11 |
| 916-923 | IVHSATGFK | A3/A11 |
| 920-928 b3a2 | ATGFKQSSK | A3/A11 |
| 924-932 b3a2 | KQSSKALQR | A3/A11 |

Suite de tableau

| | | |
|---|---|---|
| 1156-1165 | EVYEGVWKKY | A3/A11 |
| 1311-1320 | SLAYNKFS1K | A3/A11 |
| 1499-1509 | NLFSALIKK | A3/A11 |
| 1724-1734 | TVAPASGLPHK | A3/A11 |
| 1905-1914 | USTRVSLRK | A3/A11 |
| 1922-1930 | RIASGAITK | A3/A11 |
| | | |
| 924-936 b3a2 | KDSSKALDRPVAS | DR4 |

## TABLEAU 2 - Epitopes de la p53

- épitopes de la p53 se liant au HLA-A1:

RVEGNLARVEY (196-205)
GSDCTTIHY (226-234)

- épitopes de la p53 se liant au HLA- A2:

LLPENNVLSPL (25-35)
RMPEAAPPV (65-73)
RMPEAAPRV
ALNKMFCQL (129-137)
STPPPGTRV (149-157)
GLAPPQHLIRV (187-197)
LLGRNSFEV (264-272)
PLDGEYFTL (322-330)

- épitopes de la p53 se liant au HLA- A3:

RVRAMAIYK (156-164)
RRTEEENLR (282-290)
ELPPGSTKR (298-306)

- épitopes de la p53 se liant au HLA- B7:

LPENNVLSPL (26-35)
APRMPEAAPPV (63-73)
APRMPEAAPRV
APPQHLIRV (189-197)
RPILTIITL (249-257)
KPLDGETYFTL (321-330)

- épitopes de la p53 se liant au HLA- B8:

CQLAKTCPV (135-143)
GLAPPQHLI (187-195)
NTFRHSVVV (210-218)

- épitopes de la p53 se liant au HLA- B51:

LLPENNVLSPL (25-35)
RMPEAAPPV (65-73)
LIRVEGNLRV (194-203)

## TABLEAU 3 Epitopes des protéines E$_6$ et E$_7$

YMLDLQPETT (E7 11-20)
LLMGTLGIV (E7 82-90)
TLGIVCPI (E7 86-93)
TIHDIILECV (E6 29-38)

Suite de tableau

KLPQLCTEL (E6 18-26)
RPPKLPQL (E6 8-15)
LRREVYDFAFRDLCIVYRDGNPY (E6 45-67)
ISEYRHYCY (E6 80-88)
EKQRHLDKKQRFHNIRGRWT (E6 121-140)
GQAEPDRAHYNIVTF (E7 43-57)
QAEPDRAHY (E7 44-52)
EPDRAHYNIV (E7 46-55)

## TABLEAU 4: Epitopes du virus VIH-1

**HLA-A1**

(Nef 96-106: GLEGLIHSQRR
(Nef 121-128: FPDWQNYT
(Nef 137-145: LTFGWCYKL
(Nef 184-191: RFDSRLAF
(Nef 195-202: ARELHPEY

**HLA-A2**

Gp120 121-129: KLTPLCVTL
P17 77-85: SLYNTVATL
RT 200-208: ALVEICTEM
RT 275-285 VLDVGDAYFSV
RT 346-354: KRYQYMDDL
RT 368-376: KIEELRQHL
RT 376-387: LLRWGLTTPDK
RT 476-484 :ILKEPVHGV
RT 588-596: PLVKLWYQL
RT 683-692: ELVNQIIEQL
Nef 136-145: PLTFGWCFKL
Nef 180-189: VLOWRFDSRL
Nef 180-188: ALHHVAREL
Gp41 818-826: SLLNATVDI
P24 185-193: DLNTMLNTV
RT 146-354: VIYQYMDDL
RT 588-596: PLVKLWYQL
Pro 143-152: VLVGPTPVNI
(Gp120 37-44: TVYYGVPV
(Gp120 115-122: SLKPCVKL
(Gp120 313-321: RIQRGPGRA
(Gp120 197-205: TLTSCNTSV
(Gp120 428-435: FINMWQEV
(Gp41 836-844: VVQGAYRAI
(p24 219-228: HAGPIAPGQM
(p15 422-431: QMKDCTERQA
(p15 448-456: FLQSRPETA
(RT 681-691: ESELVNQIIEG

**HLA-A3**

P17 18-26: KIRLRPGGK

Suite de tableau

| HLA-A3 | |
|---|---|
| | P17 20-28: RLRPGGKKK |
| | RT 200-210: ALVEICTEMEK |
| | RT 325-333: AIFQSSMTK |
| | RT 359-368: DLEIGQHRTK |
| | Nef 73-82: QVPLRPMTYK |
| | Gp120 37-46: TVYYGVPVWK |
| | Gp41 775-785: RLRDLLLIVTR |
| | P17 18-26: KIRLRPGGK |
| HLA-A11 | |
| | RT 325-333: AJFQSSMTK |
| | RT 507-517: QIYQEPFKNLK |
| | Nef 73-82: QVPLRPMTYK |
| | Nef 84-92: AVDLSHFLK |
| | p24 349-359: ACQVGGPGHK |
| | P17 83-91: ATLYCVHQR |
| HLA-A24 (A9) | |
| | Gp120 52-61: LFCASDAKAY |
| | Gp41 591-598: YLKDQQLL |
| | ou 590-597: RYLKDQQLL |
| | (RT 484-492: VYYDPSKDL |
| | (RT 508-516: IYQEPFKNL |
| | (RT 681-691: ESELVNQIIEG |
| HLA-A25 (A10) | |
| | P24 203-212: ETINEEAAEW |
| HLA-A26 (A10) | |
| | P24 167-175: EVIPMFSAL |
| HLA-A30 (A19) | |
| | (Gp41 845-852: RAIRHIPRR |
| HLA-A31 (A19) | |
| | Gp41 775-785: RLRDLLLIVTR |
| HLA-A32 (A19) | |
| | Gp120 424-432: RIKQIINMW |
| | Gp41 774-785: HRLRDLLLI: |
| | RT 559-568: PIQKETWETW |
| HLA-A33 (A19) | |
| | (P24 266-275: IILGLNKIVR |

Suite de tableau

HLA-B7

RT 699-707: YLAWVPAHK
Nef 68-77: FPVTQVPLR
Nef 12S-137: TPGPGVRYPL
Gp120 303-312: RPNNNTRKSI
Gp41 848-856: IPRRIRQGL
RT 699-707: YLAWVPAHK

HLA-B8

Gp120 2-10: RVKEKYQHL
P17 24-32: GGKKKYKLK
Nef 90-97:FLKEKGGL =
P24 259-267: GETYKRWII
Gp41 591-598: YLKDQQLL
(Gp41 849-856: PRRIRQGL
ou 851-859: RIRQGLERIL
(P24 329-337: DCKTILKAL
(RT 185-193: GPKVKQWPL
(Nef 182-189: EWRFDSRL

HLA-B14

Gp41 589-597: ERYLKDQQL
P24 29S-306: DRFYKTLRA
(P24 183-191 ? : DLNTMLNTV
(p24 304-313: LRAEQASVQEV
(p24 305-313: RAEQASVQEV

HLA-B15

Nef 135-143: YPLTFGWCY
Nef 135-143: YPLTFGWCF

HLA-B27

P24 263-272: KRWIILGLNK
Nef 73-82: QVPLRPMTYK
Nef 134-141: RYPLTFGW
ou 133-141: YPLTFGW
Gp41 589-597. ERYLKDQQL
(Gp41 791-800: GRRGWEALKY

HLA-B35

Gp120 78-86: DPNPQEVVL
Gp120 257-265: RPWSTQLL
RT 285-294: VPLDKDFRKY
RT 323-331: SPAIFQSSM
RT 342-350: NPDIVIYQY (consensus clade B)
RT 460-468: IPLTEEAEL
RT 598-608: EPIVGAETFY
Nef 68-76: FPVRPQVPL

Suite de tableau

| HLA-B35 | |
|---|---|
| | Nef 74-81: VPLRPMTY |
| | Gp41 611.619: TAVPWNASW |
| | Gp120 42-52: VPVWKEATTTL |
| | P17 124-132: NSSOVSQNY (consensus clade B) |
| | P24 254-262: PPIPVGEIY (consensus clade B) |
| HLA-B37 | |
| | Nef 120-128: YFPDWQNYT |
| HLA-B44(B12) | |
| | P24 178-186: SEGATPQDL |
| | (p24 175-184: LESGATPQDL |
| HLA-B51 (B5) | |
| | gp41 562-570: RAIEAQQHL |
| | RT 200-208: ALVEICTEM |
| | RT 209-217: EKEGKISKI |
| | RT 295-302.: TAFTIPSI |
| HLA-B52 (B5) | |
| | Nef 190-198: AFHHVAREL |
| HLA-B55 (B22) | |
| | Gp120 42-51: VPVWKEATTTL |
| HLA-B57 et B58 (B17) | |
| | P24 240-249: TSLTQEQIGW |
| | Nef 116-125: HTQGYFPDWQ |
| | ou 116-124: HTQGYFPDW |
| | Nef 120-128: YFPDWQN |
| | (P24 147-155: ISPRTLNAW |
| | (P24 164-172: FSPEVIPMF |
| HLA-Bw62 (B15) | |
| | P17 20-29: RLRPGGKKKY |
| | P24 268-277: LGLNKIVRMY |
| | RT 427-438: LVGKLNWASQIY |
| | Nef 84-91: AVDLSHFL |
| | Nef 117-127: TQGYFPDWQNY |
| HLA-Cw4 | |
| | gp 120 380-388: SFNCGGEFF |
| HLA-Cw8 | |
| | RT 663-672: VTDSQYALGI |

Suite de tableau

HLA-Cw8

P24 305-313: RAEQASQEV

Nef 82-91: KAALDLSHPL

HLA-Cw?

P24 308-316: QATQEVKNW

**TABLEAU 5 - Epitopes de Mélanome humain**

| Gene/protéine | MHC restriction | Peptide | Position des acides aminés |
|---|---|---|---|
| Tyrosinase | HLA-A2 | MLLAVLYCL | 1-9 |
| | HLA-A2 | YMNGTMSQV | 369-377 |
| | | YMDGTMSQV | |
| | HLA-A24 | AFLPWHRLF | 206-214 |
| | HLA-B44 | SEIWRDIDF | 192-200 |
| | HLA-DR4 | QNILLSNAPLGPQFP | 56-70 |
| | | SYLQDSDPDSFQD | 450-462 |
| Pmel17gp100 | HLA-A2 | KTWGQYWQV | 154-162 |
| | HLA-A2 | AMLGTHTMEV | 177-186 |
| | HLA-A2 | MLGTHTMEV | 178-186 |
| | HLA-A2 | ITDQVPFSV | 209-217 |
| | HLA-2 | YLEPGPVTA | 280-288 |
| | HLA-A2 | ILLDGTATLRL | 457-466 |
| | HLA-A2 | VLYRYGSFSV | 476-485 |
| | HLA-A2 | SLADTNSLAV | 570-579 |
| | HLA-A3 | ALLAVGATK | 17-25 |
| Melan-AMART-1 | HLA-A2 | (E)AAGIGILTV | 26(7)-35 |
| | HLA-A2 | ILTVILGVL | 32-40 |
| gp75TRP-1 | HLA-A31 | MSLQRQFLR | |
| TRP-2 | HLA-A31 | LLGPGRPYR | 197-205 |

**TABLEAU 6: Epitopes de tumeurs résultant de mutations**

| Gene/proteine | Tumeur | MHC Restriction | Peptide | Position des acides aminés |
|---|---|---|---|---|
| MUM-1 | Mélanome | HLA-B44 | EEKLIVVLF | 30-38 |
| CDK4 | Mélanome | HLA-A2 | ACDPHSGHFV | 23-32 |
| β-catenine | Mélanome | HLA-A24 | SYLDSGIHF | 29-37 |
| HLA-A2 | carcinome rénal | - | - | - |
| CASP-8 | carcinome squameux de la tête et du cou | HLA-B35 | FPSDSWCYF | 476-484 |

**TABLEAU 7 Antigènes communs à diverses tumeurs**

| Gene | tissue où a lieu l'expression normale | MHC restriction | Peptide antigénique | Position des acides aminés |
|---|---|---|---|---|
| MAGE-1 | testicules | HLA-A1 | EADPTGHSY | 161-169 |
| | | HLA-Cw16 | SAYGEPRKL | 230-238 |

Suite de tableau

| Gene | tissue où a lieu l'expression normale | MHC restriction | Peptide antigénique | Position des acides aminés |
|---|---|---|---|---|
| MAGE-3 | testicules | HLA-A1 HLA-A2 HLA-B44 | EVDPIGHLY FLWGPRALV MEVDPIGHLY | 168-176 271-279 167-176 |
| BAGE | testicules | HLA-Cw16 | AARAVFLAL | 2-10 |
| GAGE-1/2 | testicules | HLA-Cw6 | YRPRPRRY | 9-16 |
| RAGE-1 | rétine | HLA-B7 | SPSSNRIRNT | 11-20 |
| GnTV | aucun | HLA-A2 | VLPDVFIRC | 38-64 |
| mucine | seins lors de la lactation | pas de restriction | PDTRPAPGSTAPPA HGVTSA* | |
| * transcript aberrant de la N-acétyl glucosaminyl transferase V (GnTV) trouvé uniquement dans ies mélanomes. | | | | |

**TABLEAU 8**

| LIPOPEPTIDES | RENDEMENT DE FILTRATION |
|---|---|
| NEF66 | quantitatif |
| NEF117 | 80% |
| NEF182 | quantitatif |
| GAG 183 | 80% |
| GAG253 | 77% |
| ENV | quantitatif |

**TABLEAU 9**

| 1 Peptide | Solvant | concentration (mglmL) | Volume prélevé (mL) | Rendement de filtration(%) après mélange |
|---|---|---|---|---|
| NEF66 | eau | 5 | 1 | 95 |
| NEF 117 | AcOH 25% | 5 | 1 | 81 |
| NEF 182 | AcOH 25% | 5 | 1 | 92 |
| GAG 183 | ACOH 80% | 10 | 0.5 | 73 |
| GAG 253 | AcOH 25% | 5 | 1 | 31 |
| ENV | eau | 5 | 1 | 95 |

**TABLEAU 10**

| Peptide | solvant | concentration (mg/ml) | volume prélevé (ml) | rendement de filtration (%) après mélange * |
|---|---|---|---|---|
| NEF 66 | AcOH 80% | 20 | 0,250 | quantitatif |
| NEF 117 | AcOH 80% | 20 | 0,250 | quantitatif |
| NEF 182 | AcOH 80% | 20 | 0,250 | quantitatif |
| GAG 183 | AcOH 80% | 120 | 0,250 | quantitatif |
| GAG 253 | AcOH 80% | 20 | 0,250 | quantitatif |

Suite de tableau

| Peptide | solvant | concentration (mg/ml) | volume prélevé (ml) | rendement de filtration (%) après mélange * |
|---------|---------|----------------------|---------------------|---------------------------------------------|
| ENV | AcOH 80% | 20 | 0,250 | quantitatif |
| * à la précision du dosage près. | | | | |

## TABLEAU 11

| peptide | pesée exacte*** (mg) | peptide net | quantité attendue* ($\mu$g par dose | quantité*** obtenue ($\mu$g par dose) | rendement ** (%) |
|---------|----------------------|-------------|------------------------------------|---------------------------------------|------------------|
| NEF 66 | 764 | 641 | 550 | 505+/-15 | 89.14 - 94.6 |
| NEF117 | 739 | 641 | 550 | 621+/-21 | 109.08 - 116.72 |
| NEF 182 | 742 | 641 | 550 | 545+/-16 | 96.23 - 102.05 |
| GAG183 | 741 | 642 | 550 | 478+/-13 | 84.50 - 89.23 |
| GAG253 | 780 | 641 | 550 | 571+/-28 | 98.76 - 108.95 |
| ENV | 810 | 642 | 550 | 593+/- 17 | 104.71 - 110.89 |

* la dose ciblée était de 500 $\mu$g par peptide; un surdosage de 10% a été délibérément prévu au moment de la pesée, compte tenu des rendements obtenus lors de la préparation du lot CK6.

**Les fourchettes des rendements reflètent la précision du dosage, et non une variation importante d un flacon à l'autre.

*** Les valeurs en excès sont dues aux imprécisions des pesées de poudres électrostatiques car un opérateur revêtu du scaphandre standard.

## TABLEAU 12 - Essai uniformité de teneur

| | nel 66 | nel 117 total | nel 182 | gag 183 | gag 253 | env 303 |
|---|---|---|---|---|---|---|
| | 14,40 | 17,74 | 16,19 | 13,28 | 16,74 | 17,53 |
| | 14,38 | 17,75 | 16,21 | 13,27 | 17,27 | 17,63 |
| | 14,67 | 16,36 | 16,61 | 13,41 | 16,89 | 18,36 |
| échantillon 1 | 14,49 | 17,28 | 16,34 | 13,32 | 16,97 | 17,84 |
| | 13,42 | 17,11 | 15,32 | 12,66 | 15,56 | 16,36 |
| | 13,81 | 17,04 | 15,32 | 12,67 | 15,89 | 16,51 |
| | 13,77 | 17,06 | 15,33 | 12,45 | 15,16 | 16,41 |
| échantillon 2 | 13,67 | 17,07 | 15,32 | 12,59 | 15,54 | 16,43 |
| | 13,58 | 17,08 | 15,33 | 12,68 | 15,64 | 16,29 |
| | 13,70 | 17,08 | 15,31 | 12,62 | 15,82 | 16,28 |
| | 13,59 | 17,05 | 15,31 | 12,32 | 14,85 | 16,37 |
| échantillon 3 | 13,62 | 17,07 | 15,32 | 12,54 | 15,44 | 16,31 |
| | 13,20 | 16,80 | 15,14 | 12,23 | 16,05 | 16,15 |
| | 14,53 | 17,34 | 15,74 | 13,06 | 15,93 | 17,17 |
| | 13,49 | 16,86 | 15,17 | 12,31 | 15,44 | 16,15 |
| échantillon 4 | 13,74 | 17,00 | 15,35 | 12,53 | 15,80 | 16,49 |
| | 13,88 | 17,21 | 15,40 | 12,52 | 14,78 | 16,80 |
| | 13,94 | 17,17 | 15,39 | 12,59 | 15,20 | 16,72 |
| | 13,98 | 17,19 | 15,47 | 12,96 | 15,49 | 16,71 |
| échantillon 5 | 13,94 | 17,19 | 15,42 | 12,69 | 15,16 | 16,74 |
| | 14,03 | 17,26 | 15,75 | 11,62 | 15,78 | 16,97 |
| | 13,99 | 17,20 | 15,73 | 11,39 | 15,77 | 17,02 |
| | 14,20 | 17,26 | 15,74 | 12,19 | 15,90 | 16,80 |

EP 1 035 866 B1

## TABLEAU 12 -Essai uniformité de teneur (suite)

| échantillon | | | | | | |
|---|---|---|---|---|---|---|
| échantillon 6 | 14,07 | 17,24 | 15,74 | 11,73 | 15,81 | 16,93 |
| | 13,78 | 17,29 | 15,67 | 12,69 | 16,13 | 18,04 |
| | 13,94 | 17,22 | 15,57 | 12,67 | 16,40 | 17,50 |
| | 13,95 | 17,23 | 15,55 | 12,28 | 16,19 | 17,37 |
| échantillon 7 | 13,89 | 17,25 | 15,60 | 12,55 | 16,24 | 17,64 |
| | 13,84 | 17,06 | 15,38 | 12,50 | 15,62 | 17,90 |
| | 13,65 | 17,09 | 15,45 | 12,44 | 16,02 | 17,73 |
| | 13,73 | 16,94 | 15,37 | nd | 16,21 | 17,54 |
| échantillon 8 | 13,74 | 17,03 | 15,40 | 12,47 | 15,95 | 17,73 |
| | 14,03 | 17,40 | 15,66 | 12,77 | 16,46 | 18,56 |
| | 14,07 | 17,33 | 15,72 | 11,92 | 16,61 | 18,41 |
| | 13,89 | 17,39 | 15,72 | 12,68 | 15,94 | 18,37 |
| échantillon 9 | 14,00 | 17,37 | 15,70 | 12,46 | 16,34 | 18,45 |
| | 13,34 | 16,88 | 15,33 | 12,07 | 14,70 | 17,92 |
| | 13,71 | 17,24 | 15,66 | 12,36 | 15,12 | 18,50 |
| | 13,53 | 16,93 | 15,44 | 12,28 | 14,44 | 18,01 |
| échantillon 10 | 13,53 | 17,02 | 15,48 | 12,24 | 14,76 | 18,14 |
| | 13,72 | 17,22 | 15,64 | 12,41 | 14,89 | 18,32 |
| | 13,75 | 17,33 | 15,72 | 12,36 | 14,82 | 18,31 |
| | 13,67 | 17,21 | 15,72 | 11,86 | 14,61 | 18,69 |
| échantillon 11 | 13,71 | 17,25 | 15,69 | 12,21 | 14,77 | 18,44 |
| | 13,62 | 17,11 | 15,60 | 12,28 | 14,75 | 18,42 |
| | 13,74 | 17,13 | 15,70 | 12,44 | 14,98 | 18,31 |
| | 13,75 | 17,16 | 15,63 | 12,51 | 15,37 | 18,32 |
| échantillon 12 | 13,70 | 17,13 | 15,65 | 12,41 | 15,03 | 18,35 |
| | 13,32 | 16,36 | 14,74 | 12,47 | 16,17 | 15,44 |
| | 13,31 | 16,41 | 14,68 | 12,51 | 16,26 | 15,56 |
| | 13,34 | 16,38 | 14,67 | 12,53 | 16,17 | 15,43 |

EP 1 035 866 B1

## TABLEAU 12 - Essai uniformité de teneur (suite)

| | | | | | |
|---|---|---|---|---|---|
| **échantillon 13** | 13,32 | 16,38 | 14,70 | 12,50 | 16,20 | 15,48 |
| | 13,76 | 16,72 | 14,75 | 12,67 | 16,10 | 15,59 |
| | 13,56 | 16,35 | 14,75 | 12,64 | 16,16 | 15,64 |
| | 13,60 | 16,67 | 14,76 | 12,64 | 16,06 | 15,64 |
| **échantillon 14** | 13,64 | 16,58 | 14,76 | 12,65 | 16,10 | 15,62 |
| | 13,36 | 16,40 | 14,53 | 12,48 | 15,90 | 15,64 |
| | 13,41 | 16,35 | 14,57 | 12,52 | 15,84 | 15,80 |
| | 13,44 | 16,43 | 14,50 | 12,46 | 15,77 | 15,60 |
| **échantillon 15** | 13,40 | 16,39 | 14,53 | 12,49 | 15,84 | 15,68 |
| m | 13,69 | 16,91 | 15,28 | 12,49 | 15,81 | 16,82 |
| écart type | 0,27 | 0,37 | 0,42 | 0,28 | 0,59 | 1,19 |
| t*s | 0,58 | 0,79 | 0,89 | 0,59 | 1,24 | 2,51 |
| min | 13,11 | 16,12 | 14,39 | 11,91 | 14,57 | 14,30 |
| max | 14,27 | 17,70 | 16,18 | 13,08 | 17,05 | 19,33 |
| min accepté (-15%) | 11,64 | 14,37 | 12,99 | 10,62 | 13,44 | 14,29 |
| max accepté (+15%) | 15,74 | 19,45 | 17,58 | 14,37 | 18,18 | 19,34 |
| écart observé | 4,23% | 4,67% | 5,84% | 4,70% | 7,84% | 14,95% |

t = 2,110    3 valeurs aberrantes sur échantillon 1 (erreur de dilution probable)

essai uniformité de teneur

### TABLEAU 13

| Lignées CTL anti-7 peptides | Peptides reconnus | Petits peptides reconnus |
|---|---|---|
| 92102 | GAG 2466281 | |
| 92105 | NEF 125-147 | |
| 92109 | NEF 101-126 | NEF 101-110<br>NEF 116-126 |
| | NEF 125-147 | NEF 128-136 |

Suite de tableau

| Lignées CTL anti-7 peptides | Peptides reconnus | Petits peptides reconnus |
|---|---|---|
| | NEF 155-178 | NEF 169-178 |
| | NEF 201-225 | NEF 215-225 |
| | GAG 246-281 | |
| 92120 | GAG 246-281 | |
| 92125 | NEF 155-178 | NEF 169-178 |
| 92129 | NEF 125-147 NEF 155-178 NEF 201-225 | NEF 128-136 NEF 169-178 NEF 201-211 NEF 211-219 |
| 92117 | négative | |
| 92127 | NEF 101-126 NEF 125-147 NEF 155-178 | |

**TABLEAU 14 Détection d'anticorps spécifiques de peptides des protéines NEF, GAG et ENV du virus VIH, dans le sérum de volontaires immunisés avec un mélange de six lipopeptides**

| Volontaire[a] | Période de récupération | Peptide reconnu | | | | | |
|---|---|---|---|---|---|---|---|
| | | N1 | N2 | N3 | G1 | G2 | E |
| V4.6 | W20 | 2.1 | 7 .2 | 1.0 | 1.0 | 10.2 | 1.2 |
| V4.15 | W20 | 1.3 | 4.8 | 1.2 | 1.3 | 11.2 | 1.5 |
| V4.16 | W20 | 1.2 | 4.7 | 1.2 | 1.1 | 9.7 | 1.3 |
| V4.17 | W20 | 1.7 | 1.8 | 1.0 | 1.1 | 8.0 | 1.2 |
| V4.18 | W20 | 1.1 | 1.2 | 1.0 | 1.3 | 2.2 | 1.1 |
| V4.28 | W20 | 7.8 | 8.8 | 1.5 | 1 | 15 | 5.7 |
| V4.1 (QS21) | W20 | 3.1 | 3.2 | 1.1 | 1.2 | 11.5 | 4.7 |
| V4.5 (QS21) | W20 | 1.2 | 4.2 | 1.3 | 1.1 | 8.1 | 2.1 |
| V4.19(QS21) | W20 | 1.2 | 5.3 | 1.2 | 1.3 | 5.1 | 3.8 |
| V4.21(QS21) | W20 | 1.2 | 4.7 | 1.0 | 1.2 | 9.3 | 1.9 |
| V4.32(QS21) | W20 | 6.6 | 14 | 1.8 | 1.9 | 21 | 4 |
| V4.34(QS21) | W20 | 7.1 | 21.2 | 1.2 | 1.8 | 36 | 8 |

[a] Les volontaires ont été immunises avec six lipopeptides sous forme de micelles, ou avec un adjuvant (QS21)

[b] Les sérums des volontaires ont été récupérés avant injection des lipopeptides, et vingt semaines après. Les trois injections des six lipopeptides ont été administrés à 0.4 et 16 semaines.

[c] La détection des anticorps spécifiques des peptides du virus VIH a été effectuée à l'aide d'un test ELISA avec une dilution de sérum de 1/100. Les plaques du test ELISA ont été recouvertes avec NEF66-97 (N1). NEF 117-147(N2). NEF 182-205 (N3). GAG 183-214 (G1), GAG 253-284 (G2) ou V3 ENV 303-335(E).

## TABLEAU 15
### Réponses prolifératives des PBMC des volontaires
### aux lipopeptides NEF, GAG et ENV

| Volontaire[a] | période de récupération | Index de prolifération[c] | | | |
|---|---|---|---|---|---|
| | | N1 | N2 | N3 | G1 |
| V4.6 | W0 | 1.3 | 1.0 | 1.0 | 1.3 |
| | W20 | 2.4 | 3.1(±1) | 10(±7) | 4.5(±1.1) |
| V4.15 | W0 | 1.0 | 1.0 | 1.3 | 1.0 |
| | W20 | 1.9 | 2.2 | 1.6 | 1.2 |
| V4.16 | W0 | 1.0 | 1.2 | 1.3 | 1.3 |
| | W20 | 1.1 | 1.1 | 2.2 | 4.6(± 0.6) |
| V4.17 | W0 | 2.5 | 1.3 | 1.4 | 1.9 |
| | W20 | 1.5 | 1.1 | 1.6 | 2.0 |
| V4.18 | W0 | 1.0 | 1.3 | 1.5 | 1.8 |
| | W20 | nd | nd | nd | nd |
| V4.28 | W0 | 1.3 | 2.2 | 1.7 | 1.2 |
| | W20 | 3.8(±0.6) | 1.2 | 21(±2) | 1.2 |
| | W0 | 1.0 | 1.1 | 1.0 | 1.1 |

[a] Les volontaires ont été immunisés avec six lipopeptides sous forme de micelles, ou avec un adjuvant (QS21)

[c] Les PBMC des volontaires ont été récupérées avant injection des lipopeptides (W0) et au cours de la vingtième semaine (W20).

[c] 2 10⁵ cellules ont été cultivées avec 1 µg/ml des lipopeptides du VIH et la prolifération a été mesurée par incorporation de la thymidine tritiée au jour 6. Les lipopeptides sont N1. N2. N3. G1. G2 et E. L'index de prolifération obtenu avec le milieu de culture seul est égal à 1.

[c] la réponse proliférative (cpm) des PBMC des volontaires cultivées dans le milieu seul est donnée. Tous les échantillons de PBMC ont proliféré en réponse à 1 µg/ml de PHA, PPD et SEB.

## TABLEAU 15 (suite) (1)
### Réponses prolifératives des PBMC des volontaires aux lipopeptides NEF, GAG et ENV

| Volontaire[a] | période de récupération | Index de prolifération[c] | | |
|---|---|---|---|---|
| | | G2 | E | Prolifération induite par le milieu de culture[d] |
| V4.6 | WO | 1.0 | 1 | 871 (±25) |
| | W20 | 7.2(±0.7) | 3.6(±0.9) | 280 (±32) |
| V4.15 | WO | 1.4 | 1.5 | 1657 (±182) |
| | W20 | 1.6 | 2.1 | 252 (±30) |
| V4.16 | WO | 1.8 | 1.5 | 3830 (±232) |
| | W20 | 3.6(±0.5) | 3.9(±0.7) | 1000 (±168) |
| V4.17 | WO | 2.0 | 2.3 | 5708 (±470) |
| | W20 | 2.0 | 2.8 | 1228 (±54) |
| V4.18 | WO | 3.3(±0.7) | 1.3 | 460 (±49) |
| | W20 | nd | nd | nd |
| V4.28 | WO | 1.2 | 1.2 | 869 (±36) |
| | W20 | 8.2(± 1.6) | 7.6(± 2.2) | 2558 (±186) |
| | WO | 1.0 | 1.0 | 3107 (±521) |

[a] Les volontaires ont été immunisés avec six lipopeptides sous forme de micelles. ou avec un adjuvant (QS21)

[b] Les PBMC des volontaires ont été récupérées avant injection des lipopeptides (WO) et au cours de la vingtième semaine (W20).

[c] 2 $10^5$ cellules ont été cultivées avec 1 µg/ml des lipopeptides du VIH et la prolifération a été mesurée par incorporation de la thymidine tritiée au jour 6. Les lipopeptides sont: N1. N2. N3. G1. G2 et E. L'index de prolifération obtenu avec le milieu de culture seul est égal à 1.

[d] la réponse proliférative (cpm) des PBMC des volontaires cultivées dans le milieu seul est donnée. Tous les échantillons de PBMC ont proliféré en réponse à 1 µg/ml de PHA. PPD et SEB.

## TABLEAU 15 (suite 2)
### Réponses prolifératives des PBMC des volontaires
### aux lipopeptides NEF, GAG et ENV

| Volontaire[a] | période de récupération | Index de prolifération [c] | | | |
|---|---|---|---|---|---|
| | | N1 | N2 | N3 | G1 |
| V4.1 (QS21) | W20 | nd | nd | nd | nd |
| V4.5 (QS21) | W0 | 1.5 | 1.6 | 1.5 | 1.6 |
| | W20 | 4.6(±1.2) | 3.1(±0.3) | 1.5 | 2.0 |
| V4.19 (QS21) | W0 | 1.0 | 1.1 | 1.2 | 1.1 |
| | W20 | 24.3(±3.1) | 8.5(±5) | 4.4(±1.2) | 3.1(±2.5) |
| V4.21 (QS21) | W0 | 1.3 | 1.3 | 1.2 | 3.9(±1) |
| | W20 | 6.5(±3) | 1.4 | 2.3 | 2.8 |
| V4.32 (QS21) | W0 | 1.0 | 1.0 | 1.1 | 1.3 |
| | W20 | 1.0 | 1.0 | 1.7 | 1.2 |
| V4.34 (QS21) | W0 | 1.0 | 1.1 | 1.1 | 1.2 |
| | W20 | 3.4(±0.2) | 1.1 | 3.3(±0.1) | 2.2 |

[a] Les volontaires ont été immunisés avec six lipopeptides sous forme de micelles. ou avec un adjuvant (QS21)

[c] Les PBMC des volontaires ont été récupérées avant injection des lipopeptides (W0) et au cours de la vingtième semaine (W20).

[c] 2 10[c] cellules ont été cultivées avec 1 µg/ml des lipopeptides du VIH et la prolifération a été mesurée par incorporation de la thymidine tritiée au jour 6. Les lipopeptides sont N1. N2. N3. G1. G2 et E. L'index de prolifération obtenu avec le milieu de culture seul est égal à 1.

[d] la réponse proliférative (cpm) des PBMC des volontaires cultivées dans le milieu seul est donnée. Tous les échantillons de PBMC ont proliféré en reponse à 1 µg/ml de PHA. PPD et SEB.

TABLEAU 15 (suite) (3)
Réponses prolifératives des PBMC des volontaires
aux lipopeptides NEF. GAG et ENV

| Volontaire[a] | période de récupération[b] | Index de Prolifération[c] | | |
|---|---|---|---|---|
| | | G2 | E | Prolifération induite par le milieu de culture[c] |
| V4.1 (QS21) | W20 | nd | nd | nd |
| V4.5 (QS21) | W0 | 1.5 | 1.3 | 341 (±20) |
| | W20 | 3.9(±0.3) | 5.0(±2.2) | 776 (±60) |
| V4.19 (QS21) | W0 | 1.0 | 1.0 | 918 (±102) |
| | W20 | 11.0(±2.7) | 9.4(±2.8) | 497 (±168) |
| V4.21 (QS21) | W0 | 1.3 | 1.4 | 322 (±21) |
| | W20 | 11.3(±4) | 3.3(±1.9) | 1052 (±82) |
| V4.32 (QS21) | W0 | 1.2 | 1.2 | 4448 (±75) |
| | W20 | 10.1(±1.5) | 0.9 | 245 (±30) |
| V4.34 (QS21) | W0 | 1.2 | 1.2 | 5383 (±309) |
| | W20 | 4.4(±0.6) | 3.1(±0.1) | 7381 (±280) |

[a] Les volontaires ont été immunisés avec six lipopeptides sous forme de micelles. ou avec un adjuvant (QS21)
[b] Les PBMC des volontaires ont été récupérées avant injection des lipopeptides (W0) et au cours de la vingtième semaine (W20).
[c] 2 10⁵ cellules ont été cultivées avec 1 µg/ml des lipopeptides du VIH et la prolifération a été mesurée par incorporation de la thymidine tritiée au jour 6. Les lipopeptides sont: N1. N2. N3. G1. G2 et E. L'index de prolifération obtenu avec le milieu de culture seul est égal à 1.
[c] la réponse proliférative (cpm) des PBMC des volontaires cultivées dans le milieu seul est donnée. Tous les échantillons de PBMC ont proliféré en réponse à 1 µg/ml de PHA. PPD et SEB.

## TABLEAU 16
## Spécificité des CTL chez les volontaires immunisés

% de lyse spécifique des cellules

| Lipopeptide incubé avec des cellules cibles | V4.6[a] | | V4.16 | | V4.18 | | V4.28 | |
|---|---|---|---|---|---|---|---|---|
| | W0 | W20 | W0 | W20 | W0 | W20 | W0 | W20 |
| Rapport E/T | 70/1 | | 50/1 | | 80/1 | | 10/1 | |
| Aucun | 5% | 11% | 2% | 12% | 8% | 14% | 5% | 8% |
| NEF 66-97 | 9% | 17% | 8% | 18% | 11% | 40% | 12% | 19% |
| NEF 117-147 | 9% | 16% | 2% | 13% | 6% | 6% | 19% | 12% |
| NEF 182-205 | 4% | 15% | 2% | 24% | 6% | 6% | 2% | 4% |
| Rapport E/T | 70/1 | | 30/1 | | 55/1 | | 10/1 | |
| Aucun | 4% | 18% | 5% | 5% | 8% | 6% | 2% | 2% |
| GAG 183-214 | 7% | 14% | 9% | 10% | nd | nd | 2% | 2% |
| GAG 253-284 | 9% | 49% | 11% | 20% | 6% | 26% | 2% | 2% |

[a] Les volontaires ont été immunisés avec six lipopeptides sous forme de micelles, ou avec un adjuvant (QS21)

[b] Les cellules cibles sont des PBMC autologues sensibilisées avec 10 µM de chacun des lipopeptides, irradiées et marquées a l'aide de $^{51}$Cr.

[c] Le test de relarguage du chrome a été effectué après trois stimulations in vitro. L'activité cytotoxique à l'encontre des cellules autologues EBV incubées avec des peptides ou sans peptides a été mesurée dans un test de relarguage de 4 heures. L'activité cytotoxique a été considérée comme positive quand le relarguage du chrome est supérieur de 10% à celui observé avec les cellules cibles seules. A1 et A3 correspondent à des cellules EBV incubées avec un ensemble de peptides A1 (n 137-145, n195-202, n 184-191, n121-128 pour V4.16 ou n183-191, n121-128 pour V4.28) et le peptide A3 (n73-82).

[c] Le rapport E/T (rapport cellules effectrices/cellules cibles) correspond à 5x10$^2$ cellules cibles marquées, incubées avec des quantités variables de cellules effectrices

## TABLEAU 16 (suite 1)
## Spécificité des CTL chez les volontaires immunisés

### % de lyse spécifique des cellules

| Lipopeptide incubé avec des cellules cibles | V4.5 (QS21) | | V4.19 (QS21) | | V4.21 (QS21) | | V4.34 (QS21) | |
|---|---|---|---|---|---|---|---|---|
| | WO | W20 | WO | W20 | WO | W20 | WO | W20 |
| Rapport E/T | 100/1 | | 60/1 | | 40/1 | | 35/1 | |
| Aucun | 16% | 31% | 2% | 6% | 2% | 2% | 10% | 2% |
| NEF 66-97 | 10% | 23% | 0% | 15% | 2% | 2% | 2% | 2% |
| NEF 117-147 | 18% | 47% | 2% | 27% | 2% | 2% | 2% | 2% |
| NEF 182-205 | 10% | 31% | 0% | 0% | 2% | 2% | 2% | 2% |
| Rapport E/T | 140/1 | | 60/1 | | 46/1 | | 35/1 | |
| Aucun | 23% | 11% | 0% | 0% | 2% | 2% | 2% | 2% |
| GAG 183-214 | 21% | 11% | 0% | 0% | 2% | 2% | 2% | 23% |
| GAG 253-284 | 20% | 68% | 0% | 9% | 2% | 2% | 5% | 5% |

[a] Les volontaires ont été immunisés avec six lipopeptides sous forme de micelles. ou avec un adjuvant (QS21)

[b] Les cellules cibles sont des PBMC autologues sensibilisées avec 10 µM de chacun des lipopeptides. irradiées et marquées à l'aide de $^{51}$Cr.

[c] Le test de relarguage du chrome a été effectué après trois stimulations in vitro. L'activité cytotoxique à l'encontre des cellules autologues EBV incubées avec des peptides ou sans peptides a été mesurée dans un test de relarguage de 4 neures. L'activité cytotoxique a été considérée comme positive quand le relarguage du chrome est supérieur de 10% à celui observé avec les cellules cibles seules. A1 et A3 correspondent à des cellules EBV incubées avec un ensemble de peptides A1 (n 137-145. n195-202. n 184-191. n121-128 pour V4.16 ou n183-191. n121-128 pour V4.28) et le peptide A3 (n73-82).

[d] Le rapport E/T(rapport cellules effectrices/cellules cibles) correspond à 5x10$^2$ cellules cibles marquées. incubées avec des quantités variables de cellules effectrices

## TABLEAU 16 (suite 2)
## Spécificité des CTL chez les volontaires immunisés

% de lyse spécifique des cellules

| Lipopeptide incubé avec des cellules cibles | V4.6[a] | | V4.16 | | V4.18 | | V4.28 | |
|---|---|---|---|---|---|---|---|---|
| | W0 | W20 | W0 | W20 | W0 | W20 | W0 | W20 |
| Rapport E/T | 70/1 | | 25/1 | | | | 10/1 | |
| Aucun | 3% | 6% | 32% | 23% | nd | nd | 2% | 2% |
| V3 ENV 303-335 | 2% | 36% | 12% | 49% | nd | nd | 2% | 17% |
| Rapport E/T | | | 50/1 | | | | 10/1 | |
| Aucun | | | 13% | 23% | | | 2% | 2% |
| anti-A1 | | | 2% | 48% | | | 5% | 34% |
| anti-A3 | | | nd | nd | | | nd | nd |

[a] Les volontaires ont été immunisés avec six lipopeptides sous forme de micelles. ou avec un adjuvant (QS21)

[b] Les cellules cibles sont des PBMC autologues sensibilisées avec 10 µM de chacun des lipopeptides. irradiées et marquées à l'aide de $^{51}$Cr.

[c] Le test de relarguage du chrome a été effectué après trois stimulations in vitro. L'activité cytotoxique à l'encontre des cellules autologues EBV incubées avec des peptides ou sans peptides a été mesurée dans un test de relarguage de 4 heures. L'activité cytotoxique a été considérée comme positive quand le relarguage du chrome est supérieur de 10% à celui observé avec les cellules cibles seules. A1 et A3 correspondent à des cellules EBV incubées avec un ensemble de peptides A1 (n 137-145. n195-202. n 184-191. n121-128 pour V4.16 ou n183-191. n121-128 pour V4.28) et le peptide A3 (n73-82).

[d] Le rapport E/T(rapport cellules effectrices/cellules cibles) correspond à 5x10$^3$ cellules cibles marquées. incubées avec des quantités variables de cellules effectrices

## TABLEAU 16 (suite 3)
## Spécificité des CTL chez les volontaires immunisés

## % de lyse spécifique des cellules

| Lipopeptide incubé avec des cellules cibles | V4.5 (QS21) | | V4.19 (QS21) | | V4.21 (QS21) | | V4.34 (QS21) | |
|---|---|---|---|---|---|---|---|---|
| | WO | W20 | WO | W20 | WO | W20 | WO | W20 |
| Rapport E/T | 60/1 | | 60/1 | | 86/1 | | 35/1 | |
| Aucun | 22% | 14% | 0% | 2% | 7% | 13% | 5% | 3% |
| V3 ENV 303-335 | 24% | 16% | 2% | 6% | 2% | 23% | 3% | 3% |
| Rapport E/T | | | | | | | 35/1 | |
| Aucun | | | | | | | 2% | 2% |
| anti-A1 | | | | | | | nd | nd |
| anti-A3 | | | | | | | 2% | 2% |

[a] Les volontaires ont été immunisés avec six lipopeptides sous forme de micelles, ou avec un adjuvant (QS21)

[b] Les cellules cibles sont des PBMC autologues sensibilisées avec 10 µM de chacun des lipopeptides, irradiées et marquées à l'aide de $^{51}$Cr.

[c] Le test de relarguage du chrome a été effectué après trois stimulations in vitro. L'activité cytotoxique à l'encontre des cellules autologues EBV incubées avec des peptides ou sans peptides a été mesurée dans un test de relarguage de 4 heures. L'activité cytotoxique a été considérée comme positive quand le relarguage du chrome est supérieur de 10% à celui observé avec les cellules cibles seules. A1 et A3 correspondent à des cellules EBV incubées avec un ensemble de peptides A1 (n 137-145, n195-202, n 184-191, n121-128 pour V4.16 ou n183-191, n121-128 pour V4.28) et le peptide A3 (n73-82).

[d] Le rapport E/T (rapport cellules effectrices/cellules cibles) correspond à $5 \times 10^3$ cellules cibles marquées, incubées avec des quantités variables de cellules effectrices

EP 1 035 866 B1

## TABLEAU 17

### Cellules T CD8⁺ secrétant de l'Interféron-γ /Test Ex-vivo
### Nombre de cellules secrétant de l'Interféron-γ/1 x 10⁶ cellules

| | | V4.18 A2/11 B44/60 | | V4.5 (QS21) A2/11 B18/27 | | V4.21 (QS21) A1 B8 | |
|---|---|---|---|---|---|---|---|
| | | W0 | W20 | W0 | W20 | W0 | W20 |
| HLA-A1 | NEF 121-128 | | | | | 1 | 41 |
| | NEF 137-145 | | | | | 1 | 6 |
| | NEF 184-191 | | | | | 6 | 26 |
| | NEF 195-202 | | | | | 1 | 1 |
| HLA-A2 | NEF 136-145 | 0 | 0 | 0 | 4 | | |
| | NEF 190-198 | nd | nd | 4 | 16 | | |
| | GAG 183-191 | nd | nd | 2 | 16 | | |
| HLA-A11 | NEF 73-82ᶜ | 0 | 15 | 0 | 0 | | |
| | NEF 84-92 | 0 | 55 | nd | nd | | |
| | EBN 416-424 | 500 | 500 | 66 | 63 | | |
| HLA-B8 | NEF 90-97 | | | | | 1 | 51 |
| | NEF 182-189 | | | | | 1 | 26 |
| HLA-B27 | NEF 134-141 | | | 2 | 14 | | |
| | GAG 263-272 | | | 0 | 9 | | |
| HLA-B18 | NEF 135-143 | | | 0 | 9 | | |

**Revendications**

1. Micelles mixtes ou micro-agrégats pour l'induction d'une réponse immunitaire contenant au moins:

- un premier lipopeptide comprenant au moins un épitope CTL et au moins un motif lipidique, et

- un second lipopeptide comprenant au moins un épitope T auxiliaire et au moins un motif lipidique, dont la nature peut être différente du motif du premier lipopeptide.

2. Micelles ou micro-agrégats selon la revendication 1. **caractérisés en ce que** les lipopeptides comprennent indépendamment un ou plusieurs motifs lipidiques en $C_4$ à $C_{18}$.

3. Micelles ou micro-agrégats selon l'une des revendications 1 et 2. **caractérisés en ce que** les lipopeptides comprennent indépendamment une ou deux chaînes lipidiques en $C_4$ à $C_{18}$ associées par liaison covalente à un ou deux acides aminés de la partie peptidique.

4. Micelles ou micro-agrégats selon l'une des revendications 1 à 3, **caractérisés en ce que** les motifs lipidiques des lipopeptides sont constitués de deux chaînes d'acide palmitique liées aux groupements $NH_2$ d'une lysine.

5. Micelles ou micro-agrégats selon l'une des revendications 1 à 4, **caractérisés en ce que** les motifs lipidiques des lipopeptides comprennent indépendamment un résidu d'acide palmitique, d'acide 2-amino hexadécanoïque, d'acide oléique, d'acide linoléique, d'acide linolénique de pimélautide, de triméxautide ou un dérivé du cholestérol.

6. Micelles ou micro-agregats selon l'une des revendications 1 à 5, **caractérisés en ce que** la partie non lipidique des lipopeptides, comprenant les épitopes comprend entre 10 et 100, et préférentiellement entre 10 et 50 acides aminés.

7. Micelles ou micro-agrégats selon l'une des revendications 1 à 6. **caractérisés en ce que** l'épitope T auxiliaire est un épitope multivalent.

8. Micelles ou micro-agrégats selon l'une des revendications 1 à 7. **caractérisés en ce que** l'épitope T auxiliaire est le peptide 830-843 de la toxine tétanique présentant la séquence suivante:
**QYIKANSKFIGITE**

9. Micelles ou micro-agrégats selon l'une des revendications 1 à 7. **caractérisés en ce que** l'épitope T auxiliaire est l'épitope de l'hémagglutinine ou l'épitope PADRE de séquence "AKFVAAWTLKAAA".

10. Micelles ou micro-agrégats selon l'une des revendications 1 à 9. **caractérisés en ce que** les lipopeptides contiennent au moins un épitope CTL d'une protéine spécifique du mélanome d'une protéine du VIH, du VHB, du papillomavirus, de la protéine p-53. ou d'une protéine spécifique de *Plasmodium falciparum.*

11. Micelles ou micro-agrégats selon l'une des revendications 1 à 10 **caractérisés en ce qu'**ils comprennent les lipopeptides suivants:

| | |
|---|---|
| GAG 17 | EKIRLRPGGKKKYKLKHIV K(Pam)-NH2 |
| GAG 253 | NPPIPVGEIYKRWIILGLNKIVRMYSPTSILD K(Pam)-NH2 |
| POL 325 | AIFQSSMTKILEPFRKONPDIVIYQYMDDLY K(Pam)-NH2 |
| NEF 66 | VGFPVTPQVPLRPMTYKAAVDLSHFLKEKGGL K(Pam)-NH2 |
| NEF 116 | HTQGYFPDWQNYTPGPGVRYPLTFGWLYKL K(Pam)-NH2 |
| TT | Ac-QYIKANSKFIGITELKK K(Pam)-NH2 |

dans lesquels Pam représente un motif dérivé de l'acide palmitoïque.

12. Micelles ou micro-agrégats selon l'une des revendications 1 à 10 **caractérisés en ce qu'**ils comoprennent les lipopeptides suivants:

| | |
|---|---|
| LSAE CT1 | LLSNIEEPKENIIDNLLNNIK(Pam)-$NH_2$ |
| LSA3 NRI | Ac-DELFNELLNSVDVNGEVKENILEESQK(Pam)-$NH_2$ |
| LSA3 NRII | Ac-LEESQVMDDIFNSLVKSVQQEQQHNVK(Pam)-$NH_2$ |
| LSA3 RE | K(Pam)VESVAPSVEESVAPSVEESVAENVEESVAENV-$NH_2$ |

**13.** Micelles ou micro-agrégats selon l'une des revendications 1 à 12 pour l'utilisation en tant que médicament ou vaccin.

**14.** Micelles ou micro-agrégats selon l'une des revendications 1 à 12 pour l'utilisation en tant que médicament ou vaccin à l'encontre du VIH, du VHB, du papillomavirus, de la p53, du mélanome ou de la malaria induite par *Plasmodium falciparum.*

**15.** Composition pharmaceutique **caractérisée en ce qu'**elle contient une dose pharmacologiquement efficace de micelles ou de micro-agrégats selon l'une des revendications 1 à 12 et des excipients pharmaceutiquement compatibles.

**16.** Médicament ou vaccin, **caractérisé en ce qu'**il contient des micelles ou des micro-agrégats selon l'une des revendications 1 à 12.

**17.** Procédé de fabrication de micelles ou de micro-agrégats selon l'une des revendications 1 à 12, comprenant les étapes suivantes:

- dispersion de chacun des lipopeptides les constituant dans une solution d'acide acétique concentrée à environ 80% puis
- mélange des solutions ainsi obtenues

**18.** Procédé selon la revendication 17 **caractérisé en ce que** l'obtention d'une dispersion des lipopeptides mis en solution dans l'acide acétique est vérifiée par la méthode de résonance magnétique nucléaire en deux dimensions.

**Claims**

**1.** Mixed micelles or micro-aggregates for inducing an immune response comprising at least :

- a first lipopeptide comprising at least one CTL antigenic determinant and at least one lipid unit, and
- a second lipopeptide comprising at least one helper T antigenic determinant and at least one lipid unit, which may be of a different type from the first lipopeptide unit.

**2.** Micelles or micro-aggregates according to claim 1, **characterized in that** the lipopeptides independently comprise one or more $C_4$-$C_{18}$ lipid units.

**3.** Micelles or micro-aggregates according to one of claims 1 and 2, **characterized in that** the lipopeptides independently comprise one or two $C_4$-$C_{18}$ lipid chains linked by a covalent bond to one or two amino acids of the peptide moiety.

**4.** Micelles or micro-aggregates according to one of claims 1 to 3, **characterized in that** the lipid units of the lipopeptides are made of two palmitic acid chains linked to the $NH_2$ groups of a lysine.

**5.** Micelles or micro-aggregates according to one of claims 1 to 4, **characterized in that** the lipid units of the lipopeptides independently comprise a residue of palmitic acid, 2-aminohexadecanoic acid, oleic acid, linoleic acid, linolenic acid, pimelautide, trimexautide, or a derivative of cholesterol.

**6.** Micelles or micro-aggregates according to one of claims 1 to 5, **characterized in that** the non-lipid moiety of the lipopeptides, comprising the antigenic determinants, comprises between 10 and 100, and preferably between 10 and 50 amino acids.

**7.** Micelles or micro-aggregates according to one of claims 1 to 6, **characterized in that** the helper T antigenic determinant is a multivalent antigenic determinant.

**8.** Micelles or micro-aggregates according to one of claims 1 to 7, **characterized in that** the helper T antigenic determinant is the 830-843 peptide of the tetanus toxin with the following sequence:
QYIKANSKFIGITE

**9.** Micelles or micro-aggregates according to one of claims 1 to 7, **characterized in that** the helper T antigenic determinant is the antigenic determinant of hemagglutinin or the PADRE antigenic determinant with the sequence "AKFVAAWTLKAAA".

**10.** Micelles or micro-aggregates according to one of claims 1 to 9, **characterized in that** the lipopeptides comprise at least one CTL antigenic determinant of a specific protein of melanoma, of a protein from HIV, from HBV, from papillomavirus, of protein p53, or a specific protein of *Plasmodium falciparum.*

**11.** Micelles or micro-aggregates according to one of claims 1 to 10, **characterized in that** they comprise the following lipopeptides:

| | |
|---|---|
| GAG 17 | E K I R L R P G G K K K Y K L K H I V K(Pam)-NH$_2$ |
| GAG 253 | N P P I P V G E I Y K R W I I L G L N K I V R M Y S P T S I L D K(Pam)-NH$_2$ |
| POL 325 | A I F Q S S M T K I L E P F R K Q N P D I V I Y Q Y M D D L Y K(Pam)-NH$_2$ |
| NEF 66 | V G F P V T P Q V P L R P M T Y K A A V D L S H F L K E K G G L K(Pam)-NH$_2$ |
| NEF 116 | H T Q G Y F P D W Q N Y T P G P G V R Y P L T F G W L Y K L K(Pam)-NH$_2$ |
| TT | Ac-Q Y I K A N S K F T G I T E L K K K(Pam)-NH$_2$ |

in which Pam is an unit derived of palmitoic acid.

**12.** Micelles or micro-aggregates according to one of claims 1 to 10, **characterized in that** they comprise the following lipopeptides:

| | |
|---|---|
| LSAE CT1 | L L S N I E E P K E N I I D N L L N N I K(Pam)-NH$_2$ |
| LSA3 NRI | Ac-D E L F N E L L N S V D V N G E V K E N I L E E S Q K(Pam)-NH$_2$ |
| LSA3 NRII | Ac-L E E S Q V N D D I F N S L V K S V Q Q E Q Q H N V K(Pam)-NH$_2$ |
| LSA3 RE | K(Pam) V E S V A P S V E E S V A P S V E E S V A E N V E E S V A E N V-NH$_2$ |

**13.** Micelles or micro-aggregates according to one of claims 1 to 12 for the use as a drug or a vaccine.

**14.** Micelles or micro-aggregates according to one of claims 1 to 12 for the use as a drug or a vaccine against HIV, HBV, papillomavirus, p53, melanoma or malaria induced by *Plasmodium falciparum.*

**15.** Pharmaceutical composition **characterized in that** it comprises a pharmacologically effective dose of micelles or micro-aggregates according to one of claims 1 to 12 and pharmaceutically compatible carriers.

**16.** Drug or vaccine **characterized in that** it comprises micelles or micro-aggregates according to one of claims 1 to 12.

**17.** Method for producing micelles or micro-aggregates according to one of claims 1 to 12, comprising the following steps:

- dispersion of each of the constituent lipopeptides in a solution of concentrated acetic acid of about 80% concentration then
- mixing the solutions thus obtained.

**18.** Method according to claim 17 **characterized in that** the production of a dispersion of the lipopeptides dissolved in acetic acid is controlled by the two-dimensional nuclear magnetic resonance method.

**Patentansprüche**

**1.** Gemischte Micellen oder Mikroaggregate für die Induktion einer Immunantwort, die wenigstens Folgendes enthalten:

- ein erstes Lipopeptid, das wenigstens ein CTL-Epitop und wenigstens eine Lipid-Struktureinheit umfasst; und
- ein zweites Lipopeptid, das wenigstens ein Helfer-T-Epitop und wenigstens eine Lipid-Struktureinheit, die von der Lipid-Struktureinheit des ersten Lipopeptids verschieden sein kann, umfasst.

**2.** Micellen oder Mikroaggregate gemäß Anspruch 1, **dadurch gekennzeichnet, dass** die Lipopeptide unabhängig voneinander eine oder mehrere $C_4$- bis $C_{18}$-Lipid-Struktureinheiten umfassen.

3. Micellen oder Mikroaggregate gemäß einem der Ansprüche 1 und 2, **dadurch gekennzeichnet, dass** die Lipopeptide unabhängig voneinander eine oder zwei $C_4$- bis $C_{18}$-Lipidketten umfassen, die kovalent an eine oder zwei Aminosäuren des Peptidteils gebunden sind.

4. Micellen oder Mikroaggregate gemäß einem der Ansprüche 1 bis 3, **dadurch gekennzeichnet, dass** die Lipid-Struktureinheiten der Lipopeptide aus zwei Palmitinsäureketten bestehen, die an die $NH_2$-Gruppen eines Lysinrests gebunden sind.

5. Micellen oder Mikroaggregate gemäß einem der Ansprüche 1 bis 4, **dadurch gekennzeichnet, dass** die Lipid-Struktureinheiten der Lipopeptide unabhängig voneinander einen Palmitinsäurerest, 2-Aminohexadecansäurerest, Oleinsäurerest, Linolsäurerest, Linolensäurerest, Pimelautidrest, Trimexautidrest oder ein Cholesterinderivat umfassen.

6. Micellen oder Mikroaggregate gemäß einem der Ansprüche 1 bis 5, **dadurch gekennzeichnet, dass** der Nicht-Lipid-Teil der Lipopeptide, der die Epitope umfasst, 10 bis 100 und vorzugsweise 10 bis 50 Aminosäuren umfasst.

7. Micellen oder Mikroaggregate gemäß einem der Ansprüche 1 bis 6, **dadurch gekennzeichnet, dass** das Helfer-T-Epitop ein mehrwertiges Epitop ist.

8. Micellen oder Mikroaggregate gemäß einem der Ansprüche 1 bis 7, **dadurch gekennzeichnet, dass** es sich bei dem Helfer-T-Epitop um das Peptid 830-843 des Tetanustoxins handelt, das die folgende Sequenz aufweist: QYIKANSKFIGITE.

9. Micellen oder Mikroaggregate gemäß einem der Ansprüche 1 bis 7, **dadurch gekennzeichnet, dass** es sich bei dem Helfer-T-Epitop um das Epitop von Hämagglutinin oder das Epitop PADRE mit der Sequenz "AKFVAAWTLKAAA" handelt.

10. Micellen oder Mikroaggregate gemäß einem der Ansprüche 1 bis 9, **dadurch gekennzeichnet, dass** die Lipopeptide wenigstens ein CTL-Epitop eines melanomspezifischen Proteins, eines Proteins des HIV, des HBV, des Papillomvirus, des Proteins p-53 oder eines für *Plasmodium falciparum* spezifischen Proteins enthalten.

11. Micellen oder Mikroaggregate gemäß einem der Ansprüche 1 bis 10, **dadurch gekennzeichnet, dass** sie die folgenden Lipopeptide umfassen:

| | |
|---|---|
| GAG 17 | EKIRLRPGGKKKYKLKHIV K(Pam)-NH2 |
| GAG 253 | NPPIPVGEIYKRWIILGLNKIVRMYSPTSILD K(Pam)-NH2 |
| POL 325 | AIFQSSMTKILEPFRKQNPDIVIYQYMDDLY K(Pam)-NH2 |
| NEF 66 | VGFPVTPQVPLRPMTYKAAVDLSHFLKEKGGL K(Pam)-NH2 |
| NEF 116 | HTQGYFPDWQNYTPGPGVRYPLTFGWLYKL K(Pam)-NH2 |
| TT | Ac-QYIKANSKFIGITELKK K(Pam)-NH2 |

wobei Pam eine von Palmitinsäure abgeleitete Struktureinheit darstellt.

12. Micellen oder Mikroaggregate gemäß einem der Ansprüche 1 bis 10, **dadurch gekennzeichnet, dass** sie die folgenden Lipopeptide umfassen:

| | |
|---|---|
| LSAE CT1 | LLSNIEEPKENIIDNLLNNIK(Pam)-NH$_2$ |
| LSA3 NRI | Ac-DELFNLLNSVDVNGEVKENILEESQK(Pam)-NH$_2$ |
| LSA3 NRII | Ac-LEESQVNDDIFNSLVKSVQQEQQHNVK(Pam)-NH$_2$ |
| LSA3 RE | K(Pam)VESVAPSVEESVAPSVEESVAENVEESVAENV-NH$_2$ |

13. Micellen oder Mikroaggregate gemäß einem der Ansprüche 1 bis 12 zur Verwendung als Medikament oder Impfstoff.

**14.** Micellen oder Mikroaggregate gemäß einem der Ansprüche 1 bis 12 zur Verwendung als Medikament oder Impfstoff gegen HIV, HBV, Papillomvirus, p53, Melanom oder durch *Plasmodium falciparum* verursachte Malaria.

**15.** Pharmazeutische Zusammensetzung, **dadurch gekennzeichnet, dass** sie eine pharmakologisch wirksame Dosis von Micellen oder Mikroaggregaten gemäß einem der Ansprüche 1 bis 12 und pharmazeutisch verträgliche Trägersubstanzen enthält.

**16.** Medikament oder Impfstoff, **dadurch gekennzeichnet, dass** es Micellen oder Mikroaggregate gemäß einem der Ansprüche 1 bis 12 enthält.

**17.** Verfahren zur Herstellung von Micellen oder Mikroaggregaten gemäß einem der Ansprüche 1 bis 12, das die folgenden Schritte umfasst:

- Dispergieren jedes der dieselben aufbauenden Lipopeptide in einer auf ungefähr 80% konzentrierten Essigsäurelösung; und dann
- Mischen der so erhaltenen Lösungen.

**18.** Verfahren gemäß Anspruch 17, **dadurch gekennzeichnet, dass** die Gewinnung einer Dispersion der in Essigsäure gelösten Lipopeptide durch das Verfahren der zweidimensionalen kernmagnetischen Resonanzspektroskopie (2D-NMR) überprüft wird.

FIGURE 1

FIGURE  2

# FIGURE 3

## FIGURE 4

92109

FIGURE 5A

92109

FIGURE 5B

92109

FIGURE 5C

92109

FIGURE 5D

FIGURE 5E

FIGURE 5F

FIGURE 6A

FIGURE 6B

FIGURE 6C

FIGURE 6D

FIGURE 7A

FIGURE 7B

92102    CD8=63%

**FIGURE 8**

92105    CD8=76%

**FIGURE 9**

92120

**FIGURE 10**

92125

**FIGURE 11**

Fig. 12A    Fig. 12B    Fig. 12C

% lyse spécifique

E/T    E/T    E/T

● CD8+
□ PBMC
✱ CD4+

EP 1 035 866 B1

## FIG. 13

% de lyse

70
60
50
40
30
20
10
0

16/1

☐ WT
☑ .NEF
☐ NEF-2
☐ NEF-MN
☑ NEF-A
☑ NEF-ROD

Rapport cellules effectrices/cibles